# EUROPEAN PATENT APPLICATION

(11) **EP 3 135 277 A1**
(43) Date of publication of application: **01.03.2017**
(21) Application number: 15783087.8
(22) Date of filing: 20.04.2015
(51) Int. Cl.: A61K 31/17, A61K 31/41, A61K 31/4196, A61K 31/426, A61K 31/44, A61K 31/4402, A61K 31/444, A61K 31/47, A61K 31/4709, A61K 31/505, A61K 31/506, A61P 33/00

(54) **PARASITE CONTROL AGENT OR PARASITICIDE**

(30) Priority: 22.04.2014 JP 2014088059
(71) Applicant: Nippon Soda Co., Ltd., Tokyo 100-8165 (JP)
(72) Inventor: IWASA, Takao, Odawara-shi Kanagawa 250-0280 (JP)
(74) Representative: Wills, Andrew Jonathan
(86) International application number: PCT/JP2015/061975
(87) International publication number: WO 2015/163280

(57) **Abstract**

A parasite-control or parasiticide agent that exhibis excellent effects of controlling or expelling parasites, particularly endoparasites and that can be used safely contains at least one selected from the group consisting of compounds represented by formula (I) and salts thereof as an active ingredient thereof. 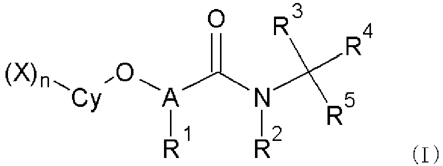 (In the formula (I), Cy represents a heteroaryl group or the like, X represents a halogeno group or the like, n is an integer of 0 to 5, in the case where n is 2 or more, X is identical or different, R¹ represents an unsubstituted or substituted C1-6 alkyl group or the like, A represents a nitrogen atom or the like, R², R³, and R⁴ each independently represents a hydrogen atom, an unsubstituted or substituted C1-6 alkyl group, or the like, and R⁵ represents an unsubstituted or substituted C6-10 aryl group, unsubstituted or substituted heteroaryl group, or the like.)

## Description

### TECHINCAL FIELD

The present invention relates to a composition useful as a medicine or an animal drug, and more specifically to a parasite control agent or parasiticide efficacious for the treatment of parasitic diseases caused by harmful endoparasites such as nematodes, cestodes, and flukes that are parasitic in humans, animals, and fish.

The present invention claims priority on the basis of Japanese Patent Application No. 2014-088059 filed in Japan on April 22, 2014, the contents of which are incorporated herein by reference.

### BACKGROUND OF THE INVENTION

Endoparasites are parasitic within the bodies of humans, animals, and fish, as hosts, and cause various parasitic diseases. Examples of the endoparasites include nematodes, cestodes, and flukes. Endoparasites are generally controlled or expelled using drugs so as to prevent ill effects. There is, however, a case where the drug becomes distributed into the surroundings and not only the animal's body, and therefore safety regarding the environment and other humans and animals is required. In addition, the problem of an increase in the number of endoparasites with drug-reistance caused by continuous administration of a single drug is a concern. Accordingly, there is a demand for a novel agent having powerful and immediate activity, long-term sustainability, and high safety for humans and animals.

Patent Documents 1, 2, and 3 disclose compounds that are harmless to fises or warm-blooded animals, but that have effects of controlling plant disease bacteria or mites that are parasitic in animals.

### DOCUMENTS OF RELATED ART

### Patent Documents

- Patent Document 1:: WO 2012/050041 A
- Patent Document 2:: WO 2013/122041 A
- Patent Document 3:: WO 2013/154080 A

### SUMMARY OF THE INVENTION

### PROBLEMS TO BE SOLVED BY THE INVENTION

An object of the present invention is to provide a parasite-control or parasiticide agent that exhibits excellent effects of controlling or expelling parasites, particularly endoparasites, and that can be safely used.

### MEANS TO SOLVE THE PROBLEMS

The present inventors have conducted studies in order to solve the above-described problems, and as a result, they have completed the present invention including the following aspects.
(1) A parasite-control or parasiticide agent containing at least one selected from the group consisting of compounds represented by formula (I) and salts thereof as an active ingredient thereof. 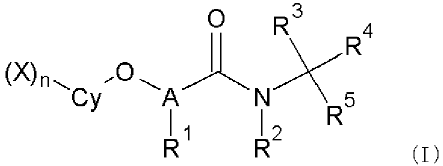
   (In the formula (I), Cy represents a C6-10 aryl group or a heteroaryl group.
   X represents a halogeno group, an unsubstituted or substituted C1-6 alkyl group, an unsubstituted or substituted C3-8 cycloalkyl group, an unsubstituted or substituted C2-6 alkenyl group, an unsubstituted or substituted C2-6 alkynyl group, a hydroxyl group, an unsubstituted or substituted C1-6 alkoxy group, an unsubstituted or substituted amino group, an unsubstituted or substituted C1-7 acyl group, an unsubstituted or substituted C1-6 alkoxycarbonyl group, an unsubstituted or substituted C1-6 alkylaminocarbonyl group, an unsubstituted or substituted C1-6 alkylthio group, an unsubstituted or substituted C1-6 alkylsulfinyl group, an unsubstituted or substituted C1-6 alkylsulfonyl group, an unsubstituted or substituted C6-10 aryl group, an unsubstituted or substituted heteroaryl group, a nitro group, or a cyano group.
   n represents the number of X on Cy and is an integer of 0 to 5. In the case where n is 2 or more, X is identical or different from each other.
   R¹ represents an unsubstituted or substituted C1-6 alkyl group, an unsubstituted or substituted C2-6 alkenyl group, or an unsubstituted or substituted C2-6 alkynyl group.
   A represents a group represented by CR^{1a} or a nitrogen atom (wherein R^{1a} represents a hydrogen atom, an unsubstituted or substituted C1-6 alkyl group, an unsubstituted or substituted C2-6 alkenyl group, or an unsubstituted or substituted C2-6 alkynyl group).
   R² represents a hydrogen atom, an unsubstituted or substituted C1-6 alkyl group, an unsubstituted or substituted C2-6 alkenyl group, an unsubstituted or substituted C2-6 alkynyl group, an unsubstituted or substituted C1-7 acyl group, or an unsubstituted or substituted C1-6 alkoxycarbonyl group.
   R³ and R⁴ each independently represents a hydrogen atom, an unsubstituted or substituted C1-6 alkyl group, an unsubstituted or substituted C2-6 alkenyl group, an unsubstituted or substituted C2-6 alkynyl group, or a cyano group.
   R⁵ represents an unsubstituted or substituted C1-6 alkyl group, an unsubstituted or substituted C2-6 alkenyl group, an unsubstituted or substituted C2-6 alkynyl group, an unsubstituted or substituted C1-7 acyl group, a carboxyl group, an unsubstituted or substituted C1-6 alkoxycarbonyl group, an unsubstituted or substituted C2-6 alkenyloxycarbonyl group, an unsubstituted or substituted C2-6 alkynyloxycarbonyl group, an aminocarbonyl group, an unsubstituted or substituted C1-6 alkylaminocarbonyl group, an unsubstituted or substituted C6-10 aryl group, or an unsubstituted or substituted heteroaryl group).
(2) The parasite-control or parasiticide agent according to (1), wherein R³ and R⁴ each independently represents an unsubstituted or substituted C1-6 alkyl group and R⁵ represents an unsubstituted or substituted C6-10 aryl group, an unsubstituted or substituted heteroaryl group, or an unsubstituted or substituted C1-6 alkylaminocarbonyl group.
(3) The parasite-control or parasiticide agent according to (1) or (2), wherein R¹ represents an unsubstituted or substituted C1-6 alkyl group, and R² represents a hydrogen atom.
(4) The parasite-control or parasiticide agent according to any one of (1) to (3), wherein A represents a nitrogen atom.
(5) The parasite-control or parasiticide agent according to any one of (1) to (3), wherein A represents a group represented by CR^{1a}, and R^{1a} represents a hydrogen atom.
(6) The parasite-control or parasiticide agent according to any one of (1) to (5), wherein the parasite-control or parasiticide agent targets an endoparasite.
(7) A method for controlling or expelling an endoparasite that is parasitic in warm-blooded animals or fish, including administering the parasite-control or parasiticide agent of (6) to the warm-blooded animals or the fish at an effective dose.
(8) The parasite-control or parasiticide agent according to (6), wherein the parasite-control or parasiticide agent targets nematodes.
(9) A method for controlling or expelling nematodes that are parasitic in warm-blooded animals or fish, comprising administering the parasite-control or parasiticide agent of (8) to the warm-blooded animals or the fish at an effective dose.

### EFFECTS OF THE INVENTION

The parasite-control or parasiticide agent according to the present invention makes it possible to effectively control or expel parasites, particularly endoparasites such as nematodes, cestodes, and flukes, that harm humans and animals.

### EMBODIMENTS FOR CARRYING OUT THE INVENTION

A parasite-control or parasiticide agent according to the present invention contains at least one selected from the group consisting of compounds represented by formula (I) (hereinafter, may be indicated as an amide compound (I)) or salts thereof as an active ingredient thereof.

### (Amide compound (I))

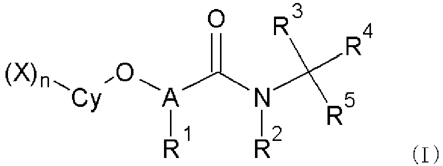

### [Substituent]

First, in the present invention, the term "unsubstituted" refers to a group consisting of a mother nucleus. In the case where only a name of a group serving as a mother nucleus is provided without being indicated with the term "substituted", this refers to "unsubstituted" unless specifically indicated otherwise.

On the other hand, the term "substituted" refers to any hydrogen atom of a group serving as a mother nucleus being substituted with a group having a structure that is the same as or different from the mother nucleus. Thus, a "substituent" is another group bound to a group serving as the mother nucleus. There may be one substituent or two or more substituents. Two or more substituents may be the same or different.

The term "C1-6", for example, indicates that the number of carbon atoms of the group serving as the mother nucleus is 1 to 6. The number of carbon atoms does not include the number of carbon atoms present in substituents. For example, a butyl group having an ethoxy group as a substituent thereof is classified as a C2 alkoxy C4 alkyl group.

There are no particular limitations on "substituents" provided that they are chemically available and achieve the effects of the present invention.

Specific examples of groups that can be "substituents" include the following groups:
halogeno groups such as a fluoro group, a chloro group, a bromo group, and an iodo group;
C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C3-8 cycloalkyl groups such as a cyclopropyl group, a cyclobutyl group, a cyclopentyl group, a cyclohexyl group, and a cycloheptyl group;
C2-6 alkenyl groups such as a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenylgroup;
C3-8 cycloalkenyl groups such as a 2-cyclopropenyl group, a 2-cyclopentenyl group, a 3-cyclohexenyl group, and a 4-cyclooctenyl group;
C2-6 alkynyl groups such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group;
C6-10 aryl groups such as a phenyl group and a naphthyl group;
C7-11 aralkyl groups such as a benzyl group and a phenethyl group;
5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group;
6-membered heteroaryl groups such as a pyridyl group, a pyrazinyl group, a pyrimidinyl group, a pyridazinyl group, and a triazinyl group;
condensed heteroaryl groups such as an indolyl group, a benzofuryl group, a benzothienyl group, a benzoimidazolyl group, a benzoxazolyl group, a benzothiazolyl group, a quinolyl group, an isoquinolyl group, and a quinoxalinyl group;
a hydroxyl group; an oxo group;
C1-6 alkoxy groups such as a methoxy group, an ethoxy group, a n-propoxy group, an i-propoxy group, a n-butoxy group, a s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
C6-10 aryloxy groups such as a phenoxy group, and 1-naphthoxy group;
C7-11 aralkyloxy groups such as a benzyloxy group, and a phenethyloxy group;
cyclic ether groups such as an oxiranyl group, a tetrahydrofuryl group, a dioxolanyl group, and a dioxlanyl group;
C1-7 acyl groups such as a formyl group, an acetyl group, a propionyl group, a benzoyl group, and a cyclohexylcarbonyl group;
C1-7 acyloxy groups such as a formyloxy group, an acetyloxy group, a propionyloxy group, a benzoyloxy group, and a cyclohexylcarbonyloxy group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
a carboxyl group;
C1-6 haloalkyl groups such as a chloromethyl group, a chloroethyl group, a trifluoromethyl group, a 1,2-dichloro-n-propyl group, 1-fluoro-n-butyl group, and a perfluoro-n-pentyl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a 2-chloro-n-propoxy group, and a 2,3-dichlorobutoxy group;
C2-6 haloalkenyloxy groups such as a 2-chloropropenyloxy group, and a 3-bromobutenyloxy group;
C1-7 haloacyl groups such as a chloroacetyl group, a trifluoroacetyl group, a trichloroacetyl group, and a 4-chlorobenzoyl group;
a cyano group; a nitro group; an amino group;
C1-6 alkylamino groups such as a methylamino group, a dimethylamino group, and a diethylamino group;
C6-10 arylamino groups such as an anilino group, and a naphthylamino group;
C7-11 aralkylamino groups such as a benzylamino group, and a phenylethylamino group;
cyclicamino groups such as an aziridinyl group, a pyrrolidinyl group, a piperidyl group, a piperazinyl group, and a morpholinyl group;
C1-7 acylamino groups such as a formylamino group, an acetylamino group, a propanoylamino group, a butyrylamino group, an i-propylcarbonylamino group, and a benzoylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group;
imino C1-6 alkyl groups such as an iminomethyl group, a (1-imino)ethyl group, and a (1-imino)-n-propyl group;
hydroxyimino C1-6 alkyl groups such as a hydroxyiminomethyl group, a (1-hydroxyimino)ethyl group, and a (1-hydroxyimino)propyl group;
C1-6 alkoxyimino C1-6 alkyl groups such as a methoxyiminomethyl group, and a (1-methoxyimino)ethyl group;
C1-6 alkoxyimino groups such as a methoxyimino group, and an ethoxyimino group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C2-6 alkenylthio groups such as a vinylthio group, and an allylthio group;
C2-6 alkynylthio groups such as an ethynylthio group, and a propargylthio group;
(C1-6 alkylthio)carbonyl groups such as a (methylthio)carbonyl group, an (ethylthio)carbonyl group, a (n-propylthio)carbonyl group, an (i-propylthio)carbonyl group, a (n-butylthio)carbonyl group, an (i-butylthio)carbonyl group, a (s-butylthio)carbonyl group, and a (t-butylthio)carbonyl group;
C1-6 alkylsulfinyl groups such as a methylsulfinyl group, an ethylsulfinyl group, and a t-butylsulfinyl group;
C2-6 alkenylsulfinyl groups such as an allylsulfinyl group;
C2-6 alkynylsulfinyl groups such as a propargylsulfinyl group;
C1-6 alkylsulfonyl groups such as a methylsulfonyl group, an ethylsulfonyl group, and a t-butylsulfonyl group;
C2-6 alkenylsulfonyl groups such as an allylsulfonyl group;
C2-6 alkynylsulfonyl groups such as a propargylsulfonyl group;
C1-6 haloalkylthio groups such as a trifluoromethylthio group, and a 2,2,2-trifluoroethylthio group;
C1-6 haloalkylsulfinyl groups such as a trifluoromethylsulfinyl group, and a 2,2,2-trifluoroethylsulfinyl group;
C1-6 haloalkylsulfonyl groups such as a trifluoromethylsulfonyl group, and a 2,2,2-trifluoroethylsulfonyl group; and
tri-C1-6 alkylsilyl groups such as a trimethylsilyl group, a triethylsilyl group, and a t-butyldimethylsilyl group.

In addition, any hydrogen atoms in these "substituents" may also be substituted with other "substituents" having a different structure.

### [Cy]

In the formula (I), Cy represents a C6-10 aryl group or a heteroaryl group.

Examples of the "C6-10 aryl group" include a phenyl group, a naphthyl group, an azulenyl group, an indenyl group, an indanyl group, and a tetralinyl group. Among these, the C6-10 aryl group is preferably a phenyl group.

The "heteroaryl group" is preferably a 5- to 10-membered aryl group that contains as constituent elements of its ring 1 to 4 heteroatoms selected from the group consisting of a nitrogen atom, an oxygen atom and sulfur atom. The heteroaryl group may be monocyclic or polycyclic. As long as at least one of the rings of a polycyclic heteroaryl group is a heteroaryl, the remaining rings may be saturated alicyclic rings, unsaturated alicyclic rings, or aromatic rings.

Examples of the "heteroaryl group" include 5-membered heteroaryl groups, 6-membered heteroaryl groups, and condensed heteroaryl groups, as exemplified as the above "substitutents". Among these, it is preferable that the heteroaryl group be a pyridyl group, a pyrimidinyl group, a pyridazinyl group, an indolyl group, a benzofuryl group, a benzothienyl group, a benzoimidazolyl group, a benzoxazolyl group, a benzothiazolyl group, a thiazolopyridyl group, a quinolyl group, an isoquinolyl group, or a quinoxalinyl group, more preferably a pyridyl group, a thiazolopyridyl group, or a quinolyl group, and even more preferably a pyridyl group.

### [X, n]

X represents a halogeno group, an unsubstituted or substituted C1-6 alkyl group, an unsubstituted or substituted C3-8 cycloalkyl group, an unsubstituted or substituted C2-6 alkenyl group, an unsubstituted or substituted C2-6 alkynyl group, a hydroxyl group, an unsubstituted or substituted C1-6 alkoxy group, an unsubstituted or substituted amino group, an unsubstituted or substituted C1-7 acyl group, an unsubstituted or substituted C1-6 alkoxycarbonyl group, an unsubstituted or substituted C1-6 alkylaminocarbonyl group, an unsubstituted or substituted C1-6 alkylthio group, an unsubstituted or substituted C1-6 alkylsulfinyl group, an unsubstituted or substituted C1-6 alkylsulfonyl group, an unsubstituted or substituted C6-10 aryl group, an unsubstituted or substituted heteroaryl group, a nitro group, or a cyano group.

n represents the number of the substituent X on Cy and is an integer of 0 to 5, and is preferably an integer of 1 to 3. In the case where n is 2 or more, X may be the same or different from each other.

Examples of the "halogeno group" as X include a fluoro group, a chloro group, a bromo group, and an iodo group.

The "C1-6 alkyl group" as X may be linear or branched. Examples of an alkyl group include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group.

Examples of the "substituted C1-6 alkyl group" include:
C1-6 haloalkyl groups such as a fluoromethyl group, a chloromethyl group, a bromomethyl group, a difluoromethyl group, a dichloromethyl group, a dibromomethyl group, a trifluoromethyl group, a trichloromethyl group, a tribromomethyl group, a 2,2,2-trifluoroethyl group, a 2,2,2-trichloroethyl group, a pentafluoroethyl group, a 4-fluorobutyl group, 4-chlorobutyl group, a 3,3,3-trifluoropropyl group, a 2,2,2-trifluoro-1-trifluoromethylethyl group, a perfluorohexyl group, a perchlorohexyl group, and a 2,4,6-trichlorohexyl group;
C3-8 cycloalkyl C1-6 alkyl groups such as a cyclopropylmethyl group, a 2-cyclopropylethyl group, a cyclopentylmethyl group, a 2-cyclohexylethyl group, and a 2-cyclooctylethyl group;
hydroxyl C1-6 alkyl groups such as a hydroxymethyl group, and a 2-hydroxyethyl group;
C1-6 alkoxy C1-6 alkyl groups such as a methoxymethyl group, an ethoxymethyl group, a methoxyethyl group, an ethoxyethyl group, a methoxy-n-propyl group, an ethoxymethyl group, an ethoxyethyl group, a n-propoxymethyl group, an i-propoxyethyl group, a s-butoxymethyl group, and a t-butoxyethyl group;
C2-6 alkenyloxy C1-6 alkyl groups such as a vinyloxymethyl group, an allyloxymethyl group, a propenyloxymethyl group, and a butenyloxymethyl group;
C6-10 aryloxy C1-6 alkyl groups such as a phenoxymethyl group;
heteroaryloxy C1-6 alkyl groups such as a pyridine-2-yl oxymethyl group;
C1-7 acyl C1-6 alkyl groups such as a formylmethyl group, an acetylmethyl group, and a propionylmethyl group;
C1-7 acyloxy C1-6 alkyl groups such as a formyloxymethyl group, an acetoxymethyl group, a 2-acetoxyethyl group, a propionyloxymethyl group, and a propionyloxyethyl group;
carboxyl C1-6 alkyl groups such as a carboxylmethyl group, and a carboxylethyl group;
C1-6 alkoxycarbonyl C1-6 alkyl groups such as a methoxycarbonylmethyl group, an ethoxycarbonylmethyl group, a n-propoxycarbonylmethyl group, and an i-propoxycarbonylmethyl group;
C1-7 acylamino C1-6 alkyl groups such as a formamidemethyl group, an acetoamidemethyl group, a 2-acetoamideethyl group, a propionylaminomethyl group, and a propionylaminoethyl group;
C1-6 alkylaminocarbonyl C1-6 alkyl groups such as a methylaminocarbonylmethyl group, an ethylaminocarbonylmethyl group, an i-propylaminocarbonylmethyl group, a t-butylaminocarbonylmethyl group, a s-butylaminocarbonylmethyl group, and a n-pentylaminocarbonylmethyl group;
C1-6 alkoxycarbonylamino C1-6 alkyl groups such as a methoxycarbonylaminomethyl group, an ethoxycarbonylaminomethyl group, an i-propoxycarbonylaminomethyl group, a t-butoxycarbonylaminomethyl group, a s-butyloxycarbonylaminomethyl group, and a n-pentyloxycarbonylaminomethyl group;
C1-6 alkoxyimino C1-6 alkyl groups such as a methoxyiminomethyl group, and a (1-methoxyimino)ethyl group,
C7-11 aralkyl groups such as a benzyl group, and a phenethyl group; and
C6-10 arylcarbonylamino C1-6 alkyl groups such as a benzoylaminomethyl group.

Examples of the "C3-8 cycloalkyl group" as X include a cyclopropyl group, a cyclobutyl group, a cyclopentyl group, a cyclohexyl group, and a cycloheptyl group.

Examples of the "C2-6 alkenyl group" as X include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "substituted C2-6 alkenyl group" include C2-6 haloalkenyl groups such as a 2-chlorol-propenyl group, and a 2-fluoro1-butenyl group.

Examples of the "C2-6 alkynyl group" as X include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "substituted C2-6 alkynyl group" include C2-6 haloalkynyl groups such as a 4,4-dichloro1-butynyl group, a 4-fluoro1-pentynyl group, and a 5-bromo-2-pentynyl group.

Examples of the "C1-6 alkoxy group" as X include a methoxy group, an ethoxy group, a n-propoxy group, a n-butoxy group, a n-pentyloxy group, a n-hexyloxy group, an i-propoxy group, an i-butoxy group, a s-butoxy group, a t-butoxy group, and an i-hexyloxy group.

Examples of the "substituted C1-6 alkoxy group" include C1-6 haloalkoxy groups such as a chloromethoxy group, a dichloromethoxy group, a difluoromethoxy group, a trichloromethoxy group, a trifluoromethoxy group, a 1-fluoroethoxy group, a 1,1-difluoroethoxy group, a 2,2,2-trifluoroethoxy group, and a pentafluoroethoxy group.

Examples of the "substituted amino group" as X include C1-6 alkylamino groups such as a methylamino group, a dimethylamino group, and a diethylamino group.

Examples of the "C1-7 acyl group" as X include a formyl group, an acetyl group, a propionyl group, and a benzoyl group.

Examples of the "substituted C1-7 acyl group" include C1-7 haloacyl groups such as a chloroacetyl group, a trifluoroacetyl group, a trichloroacetyl group, and a 4-chlorobenzoyl group.

Examples of the "C1-6 alkoxycarbonyl group" as X include a methoxycarbonyl group, an ethoxycarbonyl group, a n-propoxycarbonyl group,and an i-propoxycarbonyl group.

Examples of the "substituted C1-6 alkoxycarbonyl group" include C3-8 cycloalkyl C1-6 alkoxycarbonyl groups such as a cyclopropylmethoxycarbonyl group, a cyclobutylmethoxycarbonyl group, a cyclopentylmethoxycarbonyl group, a cyclohexylmethoxycarbonyl group, a 2-methylcyclopropylmethoxycarbonyl group, a 2,3-dimethylcyclopropylmethoxycarbonyl group, a 2-chlorocyclopropylmethoxycarbonyl group, and a 2-cyclopropylethoxycarbonyl group; and

C1-6 haloalkoxycarbonyl groups such as a fluoromethoxycarbonyl group, a chloromethoxycarbonyl group, a bromomethoxycarbonyl group, a difluoromethoxycarbonyl group, a dichloromethoxycarbonyl group, a dibromomethoxycarbonyl group, a trifluoromethoxycarbonyl group, a trichloromethoxycarbonyl group, a tribromomethoxycarbonyl group, a 2,2,2-trifluoroethoxycarbonyl group, a 2,2,2-trichloroethoxycarbonyl group, a pentafluoroethoxycarbonyl group, a 4-fluorobutoxycarbonyl group, a 3,3,3-trifluoropropoxycarbonyl group, a 2,2,2-trifluoro1-trifluoromethylethoxycarbonyl group, and a perfluorohexyloxycarbonyl group.

Examples of the "C1-6 alkylaminocarbonyl group" as X include a methylaminocarbonyl group, a dimethylaminocarbonyl group, an ethylaminocarbonyl group, and an i-propylaminocarbonyl group.

Examples of the "substituted C1-6 alkylaminocarbonyl group" include C1-6 haloalkylaminocarbonyl groups such as a fluoromethylaminocarbonyl group, a difluoromethylaminocarbonyl group, a trifluoromethylaminocarbonyl group, a 2,2,2-trifluoroethylaminocarbonyl group, a pentafluoroethylaminocarbonyl group, a 4-fluorobutylaminocarbonyl group, a 3,3,3-trifluoropropylaminocarbonyl group, a 2,2,2-trifluoro1-trifluoromethylethylaminocarbonyl group, and a perfluorohexylaminocarbonyl group.

Examples of the "C1-6 alkylthio group" as X include a methylthio group, an ethylthio group, a n-propylthio group, a n-butylthio group, a n-pentylthio group, a n-hexylthio group, and an i-propylthio group.

Examples of the "substituted C1-6 alkylthio group" include C1-6 haloalkylthio groups such as a trifluoromethylthio group, and a 2,2,2-trifluoroethylthio group.

Examples of the "C1-6 alkylsulfinyl group" as X include a methylsulfinyl group, an ethylsulfinyl group, and a t-butylsulfinyl group.

Examples of the "substituted C1-6 alkylsulfinyl group" include C1-6 haloalkylsulfinyl groups such as a trifluoromethylsulfinyl group, and a 2,2,2-trifluoroethylsulfinyl group.

Examples of the "C1-6 alkylsulfonyl group" as X include a methylsulfonyl group, an ethylsulfonyl group, and a t-butylsulfonyl group.

Examples of the "substituted C1-6 alkylsulfonyl group" include C1-6 haloalkylsulfonyl groups such as a trifluoromethylsulfonyl group, and a 2,2,2-trifluoroethylsulfonyl group.

Examples of the "C6-10 aryl group" as X include the same as those exemplified as Cy.

Examples of the "heteroaryl group" as X include the same as those exemplified as Cy.

Examples of the "substituent" on the "C6-10 aryl group" and the "heteroaryl group" as X include:
halogeno groups such as a fluoro group, a chloro group, a bromo group, and iodo group;
C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group; and
a cyano group.

X preferably represents a halogeno group, a C1-6 alkyl group, a C1-6 haloalkyl groups, a C3-8 cycloalkyl group, a C2-6 alkenyl group, a C2-6 alkynyl group, a C1-6 alkoxy group, a C1-6 haloalkoxy group, a C1-6 alkylthio group, a C1-6 haloalkylthio group, a C1-6 alkylsulfinyl group, a C1-6 haloalkylsulfinyl group, a C1-6 haloalkylsulfonyl group, a C1-6 alkylamino group, a C1-6 alkoxyimino C1-6 alkyl group, a C6-10 aryl group, a heteroaryl group, a nitro group, or a cyano group, more preferably a halogeno group, a C1-6 alkyl group, a C1-6 haloalkyl groups, a C1-6 haloalkoxy group, a C1-6 alkylamino group, a C1-6 alkoxyimino C1-6 alkyl group, a nitro group, or a cyano group.

### [R¹]

R¹ represents an unsubstituted or substituted C1-6 alkyl group, an unsubstituted or substituted C2-6 alkenyl group, or an unsubstituted or substituted C2-6 alkynyl group.

Examples of the "C1-6 alkyl group", the "C2-6 alkenyl group", and the "C2-6 alkynyl group" as R¹ include the same as those exemplified as X.

R¹ preferably represents a C1-6 alkyl group, or a C2-6 alkynyl group, more preferably a C1-6 alkyl group, and even more preferably an ethyl group.

### [A]

A represents a group represented by CR^{1a} or a nitrogen atom. R^{1a} represents a hydrogen atom, an unsubstituted or substituted C1-6 alkyl group, an unsubstituted or substituted C2-6 alkenyl group, or an unsubstituted or substituted C2-6 alkynyl group.

Examples of the "C1-6 alkyl group", the "C2-6 alkenyl group", and the "C2-6 alkynyl group" as R^{1a} include the same as those exemplified as X. R^{1a} preferably represents a hydrogen atom.

### [R²]

R² represents a hydrogen atom, an unsubstituted or substituted C1-6 alkyl group, an unsubstituted or substituted C2-6 alkenyl group, an unsubstituted or substituted C2-6 alkynyl group, an unsubstituted or substituted C1-7 acyl group, or an unsubstituted or substituted C1-6 alkoxycarbonyl group.

Examples of the "C1-6 alkyl group", the "C2-6 alkenyl group", the "C2-6 alkynyl group", the "C1-7 acyl group", and the "C1-6 alkoxycarbonyl group" as R² include the same as those exemplified as X.

R² preferably represents a hydrogen atom.

### [R³, R⁴]

R³ and R⁴ each independently represent a hydrogen atom, an unsubstituted or substituted C1-6 alkyl group, an unsubstituted or substituted C2-6 alkenyl group, an unsubstituted or substituted C2-6 alkynyl group, or a cyano group.

Examples of the "C1-6 alkyl group", the "C2-6 alkenyl group", and the "C2-6 alkynyl group" as R³ and R⁴ include the same as those exemplified as X.

R³ and R⁴ preferably represent a C1-6 alkyl group, and more preferably a methyl group.

### [R⁵]

R⁵ represents an unsubstituted or substituted C1-6 alkyl group, an unsubstituted or substituted C2-6 alkenyl group, an unsubstituted or substituted C2-6 alkynyl group, an unsubstituted or substituted C1-7 acyl group, a carboxyl group, an unsubstituted or substituted C1-6 alkoxycarbonyl group, an unsubstituted or substituted C2-6 alkenyloxycarbonyl group, an unsubstituted or substituted C2-6 alkynyloxycarbonyl group, an aminocarbonyl group, an unsubstituted or substituted C1-6 alkylaminocarbonyl group, an unsubstituted or substituted C2-6 alkynylaminocarbonyl group, an unsubstituted or substituted C6-10 aryl group, or an unsubstituted or substituted heteroaryl group.

Examples of the "C1-6 alkyl group", the "C2-6 alkenyl group", the "C2-6 alkynyl group", the "C1-7 acyl group", and the "C1-6 alkoxycarbonyl group" as R⁵ include the same as those exemplified as X.

Examples of the "C2-6 alkenyloxycarbonyl group" as R⁵ include a vinyloxycarbonyl group, an allyloxycarbonyl group, a propenyloxycarbonyl group, and a butenyloxycarbonyl group.

Examples of the "C2-6 alkynyloxycarbonyl group" as R⁵ include an ethynyloxycarbonyl group, and a propargyloxycarbonyl group.

Preferable examples of the "substituent" on the "C2-6 alkenyloxycarbonyl group" or the "C2-6 alkynyloxycarbonyl group" as R⁵ include: halogeno groups such as a fluoro group, a chloro group, a bromo group, and an iodo group; C1-6 alkylthio groups such as a methylthio group, an ethylthio group, a n-propylthio group, an i-propylthio group, a n-butylthio group, an i-butylthio group, a s-butylthio group, and a t-butylthio group; C2-6 alkynyl groups such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group; and a cyano group.

Examples of the "C1-6 alkylaminocarbonyl group" as R⁵ include a methylaminocarbonyl group, a dimethylaminocarbonyl group, an ethylaminocarbonyl group, and an i-propylcarbonyl group.

Preferable examples of the substituent on the "C1-6 alkylaminocarbonyl group" as R⁵ include: halogeno groups such as a fluoro group, a chloro group, a bromo group, and an iodo group; C1-6 alkylthio groups such as a methylthio group, an ethylthio group, a n-propylthio group, an i-propylthio group, a n-butylthio group, an i-butylthio group, a s-butylthio group, and a t-butylthio group; C2-6 alkynyl groups such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group; and a cyano group. Among these, halogeno groups, C1-6 alkylthio groups, and a cyano group are preferable, and a cyano group is more preferable.

Examples of the "C1-6 alkynylaminocarbonyl group" as R⁵ include an ethynylaminocarbonyl group, and a propargylaminocarbonyl group.

Although examples of the substituent on the "C1-6 alkynylaminocarbonyl group" include the same as those exemplified as the substituent on the "C1-6 alkylaminocarbonyl group", it is preferable that the C1-6 alkynylaminocarbonyl group be unsubstituted.

Examples of the "C6-10 aryl group" as R⁵ include a phenyl group, a naphthyl group, an azulenyl group, an indenyl group, an indanyl group, and a tetralinyl group, and a phenyl group is preferable.

Examples of the "heteroaryl group" as R⁵ include:
5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group;
6-membered heteroaryl groups such as a pyridyl group, a pyrazinyl group, a pyrimidinyl group, a pyridazinyl group, and a triazinyl group; and
condensed heteroaryl groups such as an indolyl group, a benzofuryl group, a benzothienyl group, a benzoimidazolyl group, a benzoxazolyl group, a benzothiazolyl group, a quinolyl group, an isoquinolyl group, and a quinoxalinyl group. Among these, a pyrazolyl group, a thiazolyl group, a triazolyl group, a pyridyl group, a pyrazinyl group, a pyrimidinyl group, or a triazinyl group is preferable.

It is preferable that the aryl group and the heteroaryl group be unsubstituted or substituted with 1 to 4 substituents. In the case where the groups have two or more substituents, the substituents may be the same as or different from each other.

Examples of the substituent on the aryl group and the heteroaryl group as R⁵ include the following substituents:
halogeno groups such as a fluoro group, a chloro group, a bromo group, and an iodo group;
C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C3-8 cycloalkyl groups such as a cyclopropyl group, a cyclobutyl group, a cyclopentyl group, a cyclohexyl group, and a cycloheptyl group;
C2-6 alkenyl groups such as a vinyl group, a 1-propenyl group, a 2-propenyl group, a 1-butenyl group, a 2-butenyl group, a 3-butenyl group, a 1-methyl2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenylgroup;
C2-6 alkynyl groups such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group;
C1-6 alkoxy groups such as a methoxy group, an ethoxy group, a n-propoxy group, an i-propoxy group, a n-butoxy group, a s-butoxy group, an i-butoxy group, and a t-butoxy group;
C1-6 haloalkoxy groups such as a 2-chloro-n-propoxy group, and a 2,3-dichlorobutoxy group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group, and a 2,2,2-trifluoroethylsulfinyl group;
C1-6 alkylsulfonyl groups such as a methylsulfonyl group, an ethylsulfonyl group, an i-propylsulfonyl group, and a t-butylsulfonyl group;
C1-6 haloalkylsulfonyl groups such as a trifluoromethylsulfonyl group, and a 2,2,2-trifluoroethylsulfonyl group;
C1-6 alkylamino groups such as a methylamino group, an ethylamino group, a dimethylamino group, and a diethylamino group;
C6-10 aryl groups such as a phenyl group, a naphthyl group, and a tolyl group;
5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, and a thiophenyl group;
6-membered heteroaryl groups such as a pyridyl group, a pyrazinyl group, a pyrimidinyl group, a pyridazinyl group, and a triazinyl group;
C1-6 alkylsulfoximino groups, such as a S,S-dimethylsulfoximino group;
a cyano group;
C3-8 cycloalkenyl groups such as a 2-cyclopropenyl group, a 2-cyclopentenyl group, a 3-cyclohexenyl group, and a 4-cyclooctenyl group;
C7-11 aralkyl groups such as a benzyl group, and a phenethyl group;
a hydroxyl group; an oxo group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
C6-10 aryloxy groups such as a phenoxy group, and a 1-naphthoxy group;
C7-11 aralkyloxy groups such as a benzyloxy group, and a phenethyloxy group;
cyclic ether groups such as an oxiranyl group, a tetrahydrofuryl group, a dioxolanyl group, and a dioxlanyl group;
C1-7 acyl groups such as a formyl group, an acetyl group, a propionyl group, a benzoyl group, and a cyclohexylcarbonyl group;
C1-7 acyloxy groups such as a formyloxy group, an acetyloxy group, a propionyloxy group, a benzoyloxy group, and a cyclohexylcarbonyloxy group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
a carboxyl group;
C1-7 haloacyl groups such as a chloroacetyl group, a trifluoroacetyl group, a trichloroacetyl group, and a 4-chlorobenzoyl group;
a nitro group; an amino group;
C6-10 arylamino groups such as an anilino group, and a naphthylamino group;
C7-11 aralkylamino groups such as a benzylamino group, and a phenylethylamino group;
cyclicamino groups such as an aziridinyl group, a pyrrolidinyl group, a piperidyl group, a piperazinyl group, and a morpholinyl group;
a hydrazino group; C1-6 alkylhydrazino groups such as a N-methylhydrazino group, and N,N'-dimethylhydrazino group;
C1-7 acylamino groups such as a formylamino group, an acetylamino group, a propanoylamino group, a butyrylamino group, an i-propylcarbonylamino group, and a benzoylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group; and
a mercapto group.

It is preferable that the "substituent on the aryl group and the heteroaryl group as R⁵" be a halogeno group, a C1-6 alkyl group, a C1-6 haloalkyl groups, a C3-8 cycloalkyl group, a C2-6 alkenyl group, a C2-6 alkynyl group, a C1-6 alkoxy group, a C1-6 haloalkoxy group, a C1-6 alkylthio group, a C1-6 haloalkylthio group, a C1-6 alkylsulfinyl group, a C1-6 haloalkylsulfinyl group, a C1-6 alkylsulfonyl group, a C1-6 alkylamino group, a C6-10 aryl group, a 5- to 6-membered heteroaryl group, a C1-6 alkylsulfoximino group, a C1-6 alkylhydrazino group, or a cyano group, and more preferably a halogeno group, a C1-6 alkyl group, a C1-6 haloalkyl groups, a C1-6 haloalkoxy group, a C1-6 alkylthio group, a C1-6 haloalkylthio group, a C1-6 alkylsulfinyl group, a C1-6 haloalkylsulfinyl group, a C1-6 alkylsulfonyl group, a C1-6 alkylamino group, a C6-10 aryl group (more preferably a phenyl group), a 5- to 6-membered heteroaryl group (more preferably an imidazolyl group, a thiophenyl group, a pyridyl group, or a pyrazinyl group), a C1-6 alkylsulfoximino group, or a C1-6 alkylhydrazino group.

It is preferable that R⁵ represent an unsubstituted or substituted C6-10 aryl group, an unsubstituted or substituted heteroaryl group, or an unsubstituted or substituted C1-6 alkylaminocarbonyl group, more preferably an unsubstituted or substituted heteroaryl group, and even more preferably an unsubstituted or substituted 6-membered heteroaryl group.

### (Salt of amide compound)

A salt of the amide compound (I) is not particularly limited provided that the salt is chemically acceptable. Examples of the salt include: salts of an inorganic acid such as hydrochloric acid or sulfuric acid; salts of an organic acid such as acetic acid or lactic acid; salts of an alkali metal such as lithium, sodium, or potassium; salts of an alkali earth metal such as calcium, magnesium, or barium; salts of a transition metal such as iron, copper, or silver; and salts of an organic base such as ammonia, triethylamine, tributylamine, pyridine, or hydrazine. The salt of the amide compound (I) may be prepared by a conventional method from the amide compound (I).

Specific examples and preparation methods of the amide compound (I) or the salt thereof are described in Patent Documents 1, 2, and 3. Although the amide compound (I) or the salt thereof may be easily prepared or obtained by referring to the documents, one embodiment thereof is shown below.

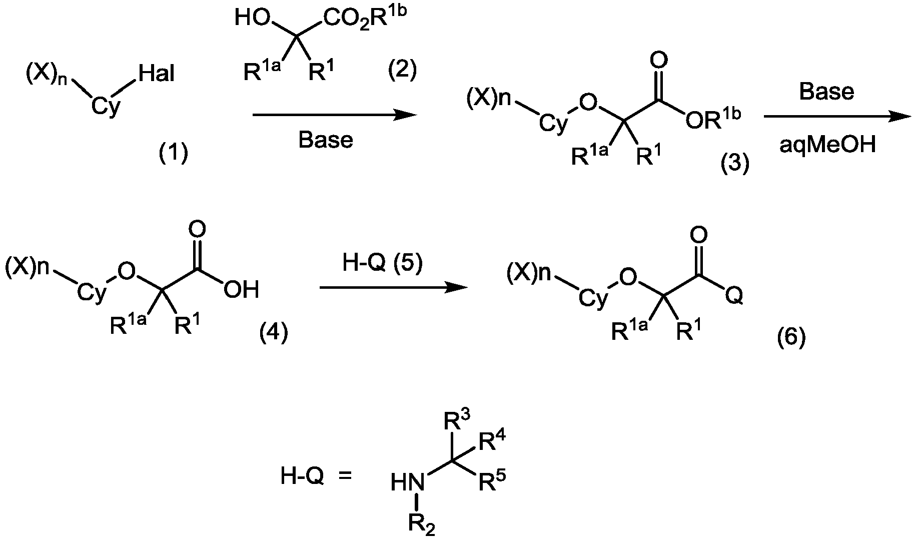

A haloaryl compound represented by formula (1) is reacted with an ester compound represented by formula (2) in the presence of a base to obtain an aryloxy acetic acid ester compound represented by formula (3). Then, the aryloxy acetic acid ester compound is hydrolyzed to obtain a compound represented by formula (4).

The compound (4) is reacted with a compound represented by formula (5) to obtain a compound represented by formula (6).

In the formulae (1), (2), (3), (4), and (5), X, n, R¹, R^{1a}, R² to R⁵, and Cy represent the same as those mentioned above. In the formula (1), Hal represents a halogen atom. In the formulae (2) and (3), R^{1b} represents a C1-6 alkyl group.

A compound represented by the following formula (II) is a novel compound.

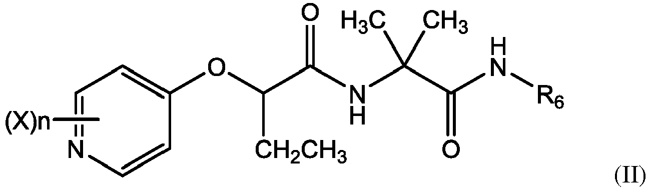

(In the formula (II), R₆ represents a C1-6 haloalkyl group, and X and n represent the same as those mentioned above.)

Parasites that are targets of the parasite-control or parasiticide agent according to the present invention are parasitic in host animals, particularly in warm-blooded animals or fish (endoparasites). Examples of the host animals on which the parasite-control or parasiticide agent according to the present invention is effective include: warm-blooded animals such as humans, domestic mammals (such as cows, horses, pigs, sheeps, and goats), laboratory animals (such as mice, rats, and jirds), pet animals (such as hamsters, guinea pigs, dogs, cats, horses, squirrels, rabbits, and ferrets), mammals in nature or zoo (such as monckies, foxes, deers, and buffalos), poultry (such as turkeis, ducks, chickens, quails, and geese), and pet birds (such as pigeons, parrots, magpies, java sparrows, parakeets, bengalees, and canaries); and fises such as salmon, trout, and koi carp. It is possible to prevent or treat parasitic diseases mediated by parasites by controlling or expelling the parasites.

Examples of the parasite targeted to be controlled or expelled include the followings.
(1) Nematodes of the Enoplida Order:
   (a) kidney worms belonging to the Dioctophymatidae family, such as Dioctophyma renale of Dioctophyma spp.; and
   (b) kidney worms belonging to the Soboliphymatidae family, such as Soboliphyme abei and Soboliphyme baturini of Soboliphyme spp.
(2) Nematodes of the Enoplida Order:
   (a) trichinae belonging to the Trichinellidae family, such as Trichinella spiralis of Trichinella spp.; and
   (b) whipworms belonging to the Trichuridae family, such as Capillaria annulata, Capillaria contorta, Capillaria hepatica, Capillaria perforans, Capillaria plica, and Capillaria suis, of Capillaria spp.; and Trichuris vulpis, Trichuris discolor, Trichuris ovis, Trichuris skrjabini, and Trichuris suis, of Trichuris spp.
(3) Nematodes of the Rhabditida Order:
   strongyloides stercoralis belonging to the Strongyloididae family, such as Strongyloides papillosus, Strongyloides planiceps, Strongyloides ransomi, Strongyloides suis, Strongyloides stercoralis, Strongyloides tumefaciens, and Strongyloides ratti, of Strongyloides spp.
(4) Nematodes of the Strongylida Order (Strongylida):
   ucinarias belonging to the Ancylostomatidae family, such as Ancylostoma braziliense, Ancylostoma caninum, Ancylostoma duodenale, and Ancylostoma tubaeforme, of Ancylostoma spp.; Uncinaria stenocephala of Uncinaria spp.; and Bunostomum phlebotomum, and Bunostomum trigonocephalum, of Bunostomum spp.
(5) Nematodes of the Strongylida Order:
   (a) nematodes belonging to the Angiostrongylidae family, such as Aelurostrongylus abstrusus of Aelurostrongylus spp.; and Angiostrongylus vasorum, and Angiostrongylus cantonesis, of Angiostrongylus spp.;
   (b) nematodes belonging to the Crenosomatidae family, such as Crenosoma aerophila, and Crenosoma vulpis, of Crenosoma spp.;
   (c) nematodes belonging to the Filaroididae family, such as Filaroides hirthi, and Filaroides osleri, of Filaroides spp.;
   (d) metastrongyles belonging to the Metastrongylidae family, such as Metastrongylus apri, Metastrongylus asymmetricus, Metastrongylus pudendotectus, and Metastrongylus salmi, of Metastrongylus spp.; and
   (e) gapeworms belonging to the Syngamidae family, such as Cyathostoma bronchialis of Cyathostoma spp.; and Syngamus skrjabinomorpha, and Syngamus trachea, of Syngamus spp.
(6) Nematodes of the Strongylida Order:
   (a) nematodes belonging to the Molineidae family, such as Nematodirus filicollis, and Nematodirus spathiger, of Nematodirus spp.;
   (b) nematodes belonging to the Dictyocaulidae family, such as Dictyocaulus filaria, and Dictyocaulus viviparus, of Dictyocaulus spp.;
   (c) nematodes belonging to the Haemonchidae family, such as Haemonchus contortus of Haemonchus spp.; and Mecistocirrus digitatus of Mecistocirrus spp.;
   (d) nematodes belonging to the Haemonchidae family, such as Ostertagia ostertagi of Ostertagia spp.;
   (e) nematodes belonging to the Heligmonellidae family, such as Nippostrongylus braziliensis of Nippostrongylus spp.; and
   (f) nematodes belonging to the Trichostrongylidae family, such as Trichostrongylus axei, Trichostrongylus colubriformis, and Trichostrongylus tenuis, of Trichostrongylus spp.; Hyostrongylus rubidus of Hyostrongylus spp.; and Obeliscoides cuniculi of Obeliscoides spp.
(7) Nematodes of the Strongylida Order:
   (a) nematodes belonging to the Chabertiidae family, such as Chabertia ovina of Chabertia spp.; and Oesophagostomum brevicaudatum, Oesophagostomum columbianum, Oesophagostomum dentatum, Oesophagostomum georgianum, Oesophagostomum maplestonei, Oesophagostomum quadrispinulatum, Oesophagostomum radiatum, Oesophagostomum venulosum, and Oesophagostomum watanabei, of Oesophagostomum spp.;
   (b) nematodes belonging to the Stephanuridae family, such as Stephanurus dentatus of Stephanurus spp.; and
   (c) nematodes belonging to the Strongylidae family, such as Strongylus asini, Strongylus edentatus, Strongylus equinus, and Strongylus vulgaris, of Strongylus spp.
(8) Nematodes of the Oxyurida Order:
   nematodes belonging to the Oxyuridae family, such as Enterobius anthropopitheci, and Enterobius vermicularis, of Enterobius spp.; Oxyuris equi of Oxyuris spp.; and Passalurus ambiguus of Passalurus spp.
(9) Nematodes of the Ascaridida Order:
   (a) nematodes belonging to the Ascaridiidae family, such as Ascaridia galli of Ascaridia spp.;
   (b) nematodes belonging to the Heterakidae family, such as Heterakis beramporia, Heterakis brevispiculum, Heterakis gallinarum, Heterakis pusilla, and Heterakis putaustralis, of Heterakis spp.;
   (c) nematodes belonging to the Anisakidae family, such as Anisakis simplex of Anisakis spp.;
   (d) nematodes belonging to the Ascarididae family, such as Ascaris lumbricoides, and Ascaris suum, of Ascaris spp.; and Parascaris equorum of Parascaris spp.; and
   (e) nematodes belonging to the Toxocaridae family, such as Toxocara canis, Toxocara leonina, Toxocara suum, Toxocara vitulorum, and Toxocara cati, of Toxocara spp.
(10) Nematodes of the Spirurida Order
   (a) nematodes belonging to the Onchocercidae family, such as Brugia malayi, Brugia pahangi, and Brugia patei, of Brugia spp.; Dipetalonema reconditum of Dipetalonema spp.; Dirofilaria immitis of Dirofilaria spp.; Filaria oculi of Filaria spp.; and Onchocerca cervicalis, Onchocerca gibsoni, and Onchocerca gutturosa, of Onchocerca spp.;
   (b) nematodes belonging to the Setariidae family, such as Setaria digitata, Setaria equina, Setaria labiatopapillosa, and Setaria marshalli, of Setaria spp.; and Wuchereria bancrofti of Wuchereria spp.; and
   (c) nematodes belonging to the Filariidae family, such as Parafilaria multipapillosa of Parafilaria spp.; and Stephanofilaria assamensis, Stephanofilaria dedoesi, Stephanofilaria kaeli, Stephanofilaria okinawaensis, and Stephanofilaria stilesi of Stephanofilaria spp.
(11) Nematodes of the Spirurida Order:
   (a) nematodes belonging to the Gnathostomatidae family, such as Gnathostoma doloresi, and Gnathostoma spinigerum, of Gnathostoma spp.;
   (b) nematodes belonging to the Habronematidae family, such as Habronema majus, Habronema microstoma, and Habronema muscae, of Habronema spp.; and Draschia megastoma of Draschia spp.;
   (c) nematodes belonging to the Physalopteridae family, such as Physaloptera canis, Physaloptera cesticillata, Physaloptera erdocyona, Physaloptera felidis, Physaloptera gemina, Physaloptera papilloradiata, Physaloptera praeputialis, Physaloptera pseudopraerutialis, Physaloptera rara, Physaloptera sibirica, and Physaloptera vulpineus, of Physaloptera spp.;
   (d) nematodes belonging to the Gongylonematidae family, such as Gongylonema pulchrum of Gongylonema spp.;
   (e) nematodes belonging to the Spirocercidae family, such as Ascarops strongylina of Ascarops spp.; and
   (f) nematodes belonging to the Thelaziidae family, such as Thelazia callipaeda, Thelazia gulosa, Thelazia lacrymalis, Thelazia rhodesi, and Thelazia skrjabini, of Thelazia spp.

The parasite-control or parasiticide agent according to the present invention contains as active ingredients only one kind or two or more kinds of the compound according to the present invention. In addition, the parasite-control or parasiticide agent may contain conventional antiparasitic agents, insecticides, or acaricides, as additional active ingredients. Specificic examples thereof include the followings.
(1) Abamectin-based: abamectin, emamectin benzoate, lepimectin, milbemectin; ivermectin, selamectin, doramectin, eprinomectin, moxidectin, milbemycin, milbemycin oxime.
(2) Benzimidazole-based: fenbendazole, albendazole, triclabendazole, oxybendazole, mebendazole, oxfendazole, parbendazole, flubendazole; febantel, netobimin, thiophanate; thiabendazole, cambendazole.
(3) Salicylanilide-base: closantel, oxyclozanid, rafoxanide, niclosamide.
(4) Substituted phenol-based: nitroxinil, nitroscanate.
(5) Pyrmidine-based: pyrantel, morantel.
(6) Imidazothiazole-based: levamisole, tetramisole.
(7) Tetrahydropyrmidine-based: praziquantel, epsiprantel.
(8) Latrophilin receptor agonists: depsipeptide, cyclic depsipeptide, 24-membered cyclic depsipeptide, emodepside.
(9) Other antiparasitic agents: cyclodiene, ryania, clorsulon, metronidazole, demiditraz; piperazine, diethylcarbamazine, dichlorophene, phenothiazine, monepantel, tribendimidine, derquantel, amidantel; thiacetarsamide, melorsamine, arsenamide;
(10) Organophosphate-based acetylcholinesterase inhibitors:
   chlorpyriphos, diazinon, phosmet, tetrachlorovinphos, trichlorfon, haloxon, dichlorvos, naphthalophos;
(11) GABAergic chloride ion channel antagonists: chlordene, endosulfan, ethiprole, fipronil, pyrafluoprole, pyriprole; camphlechlor, heptachlor.
(12) Sodium channel modulators: bifenthrin, beta-cyfluthrin, cyhalothrin, gamma-cyhalothrin, cypermethrin, deltamethrin, esfenvalerate, fenpropathrin, fenvalerate, flucythrinate, tau-fluvalinate, permethrin, tefluthrin, tralomethrin, pyrethrin, profluthrin, dimefluthrin, metofluthrin, protrifenbute.
(13) Nicotinic acetylcholine receptor agonists: acetamiprid, clothianidin, dinotefuran, imidacloprid, nitenpyram, thiacloprid, thiamethoxam, sulfoxaflor;
(14) Nicotinic acetylcholine receptor allosteric modulators: spinetoram, spinosad;
(15) Juvenile hormone mimics: methoprene, fenoxycarb, pyriproxyfen.
(16) Homopteran selective feeding inhibitors: flonicamid, pymetrozine, pyrifluquinazon;
(17) Mite growth inhibitors: clofentezine, diflovidazin, hexythiazox, etoxazole;
(18) Mitochondrial ATP biosynthetic enzyme inhibitors: diafenthiuron.
(19) Oxidative phosphorylation uncoupling agent: chlorfenapyr.
(20) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezine.
(21) Dipteran Molting disrupting compounds: cyromazine.
(22) Molting hormone receptor agonists: chromafenozide, halofenozide, methoxyfenozide, tebufenozide;
(23) Octopamine receptor agonists: amitraz, demiditraz, chlordimeform.
(24) Mitochondrial transport system complex I inhibitors: tebufenpyrad, tolfenpyrad, rotenone.
(25) Voltage-dependent sodium channel blockers: indoxacarb, metaflumizone.
(26) Acetyl-CoA carboxylase inhibitors: spirodiclofen, spiromesifen, spirotetramat.
(27) Mitochondrial transport system complex II inhibitors: cyflumetofen.
(28) Ryanodine receptor modulators: chlorantraniliprole, cyantraniliprole, flubendiamide, cyclaniliprole, tetraniliprole;
(29) Mixed function oxidase inhibitors: piperonyl butoxide.
(30) Succinate dehydrogenase inhibitors: benodanil, flutolanil, mepronil, isofetamide, fluopyram, fenfuram, furmecyclox, carboxin, oxycarboxin, thifluzamide, benzovindiflupyr, bixafen, fluxapyroxad, furametpyr, isopyrum, penflufen, penthiopyrad, sedaxane, boscalid;
(31) Other agents: azadirachtin, bifenazate, pyridalyl, pyrifluquinazon; amidoflumet, tetramethylfluthrin, broflanilide, afoxolaner, fluralaner;

The parasite-control or parasiticide agent according to the present invention may be applied so that the compound according to the present invention is applieid at a does (medicinally effective dose) of 0.01 to 1000 mg per 1kg of host animal. The parasite-control or parasiticide agent according to the present invention may be applied using conventional medicinal or vertinary techniques (local, oral, parenteral, or subcutaneous administration). Examples thereof include: methods of orally administering tablets, capsules, or feed additives to animals; methods of administering immersion liquid, suppository, or injection (such as intramuscular, subcutaneous, intravenous, or intraabdomnal injection) to animals; methods of locally administering oily or aqueous liquid formulation by conducting spraying, pouring on, or spotting on; and methods of local administration in which the parasite-control or parasiticide agent is kneaded into a resin, the kneaded product is molded into a suitable shape such as a collar or an ear tag, and then the resultant is attached to animals.

Preferable administration methods to the host animals include oral, subcutaneous, or intravenous administration.

The parasite-control or parasiticide agent according to the present invention may contain only the compound according to the present invention, or further contain a carrier such as a liquid carrier, a gaseous carrier, or a solid carrier, and a surfactant or other auxiliary agent, as needed, to obtain formuation depending on the intended purpose. The parasite-control or parasiticide agent according to the present invention may be impregnated with a substrate such as a porous ceramic plate or nonwoven fabric.

The parasite-control or parasiticide agent according to the present invention is not particularly Imited by the dosage form. Examples of the dosage form include powders, granules, powdered agents, capsules, premixes, liquid agents, emulsions, suspensions, wafers, biscuits, and minced meats.

Examples of the dosage form suitable to oral administration include: liquid formulations such as liquid medicinal formulations and drinking water formulations; pasty or gel-like semi-solid formulations; and solid formulations such as tables, capsules, granules, and premixes. The liquid formulations may be directly administerd to the mouth or throat of the aminal using a liquid medicinal gun, a syringe, or another suitable apparatus. The semi-solid formulation may be directly administerd to the mouth of the animal using an applicator or mixed with feed to be administered. The solid formulation may be directly administered to the animal or mixed with feed to be administered.

It is possible to add a surfactant to the formulation so as to make the formulation uniform and stable. Examples of the surfactant incude: non-ionic surfactants such as polyoxyethylene-added alkylethers, polyoxyethylene-added higher fatty acid esters, polyoxyethylene-added sorbitan higher fatty acid esters, and polyoxyethylene-added tristyrylphenyl ethers; sulfate ester salts of polyoxyethylene-added alkylphenylethers, alkylnaphthalenesulfonates, polycarboxylates, lignin sulfonates, formaldehyde condensates of alkylnaphthalenesulfonates, and copolymers of isobutylene - maleic anhydride.

Among the liquid formulations, a pour-on formulation or a spot-on formulation appropriately contains a liquid carrier generally regarded as a spreading agent that promotes rapid dispersion onto the skin surface or into the fur of the host animal.

Preferable examples of the liquid carrier include: alcohols such as isopropanol, 2-octyldodecanol, oleyl alcohol, and benzyl alcohol; glycols such as diethylene glycol and ethyl carbitol; long-chain fatty acid esters such as isopropyl myristate, isopropyl palmitate, decyl oleate, hexyl larurate, oleyl oleate, decyl oleate, and capric acid esters of C12-18 alkanols; dicarboxylic acid esters such as dibutyl phthalate, diisopropyl isophthalate, diisopropyl adipate, and di-n-butyl adipate; and cyclic amides such as pyrrolidones and NMP.

Additional examples thereof include: vegetable oils such as olive oil, peanut oil, sesame oil, pine oil, linseed oil, and castor oil; paraffin, and silicone oil.

Examples of the gaseous carrier to be used to prepare a spray agent include butane, LPG, dimethyl ether, and carbon dioxide.

Examples of the solid carrier or an additive to be used to prepare a solid formulation include: vegetable powders such as cellulose derivatives such as hydroxypropyl cellulose, and carboxymethyl cellulose, lactose, sucrose, glucose, starch, flour, corn flour, soybean oil cake, defatted rice bran, soybean grain, and wheat flour; other commercially available feed materials; mineral fine powders such as diatomaceous earth, apatite, gypsum, talc, bentonite, pyrophyllite, and clay; and organic or inorganic compounds such as calcium carbonate, sodium benzoate, urea, and salt cake.

In addition, various kinds of components other than the above-mentioned components may be contained. Examples thereof include various vitamins, minerals, hormones, amino acids, enzyme formulations, antipyretics, sedatives, antiphlogistics, anti-cancer agents, antibiotics, antibacterial agents, fungicides, colorants, fragrances, preservatives, and vaccine.

An injection formulation may be prepared using a solvent, a solubilizer, a protective agent, a dispersing agent, a wetting agent, a suspending agent, and/or the like, in accordance with a conventional technique. Examples of a carrier material include water, ethanol, butanol, benzyl alcohol, glycerine, 1,3-butanediol, Ringer's solution, isotonic sodium chloride solution, bland fixed oils, vegetable oils, dextrose, mannitol, fatty acids, dimethyl acetamide, surfactant, N- methylpyrrolidone, and propylene glycol. Examples of the solubilizer include polyvinylpyrrolidone. Examples of the protective agent include benzyl alcohol, and trichloro butanol.

Although some formulations of the parasite-control or parasiticide agent according to the present invention are shown, additives and the addition ratio are not intended to be limited thereto, and may be modified widely. The term "part" in the formulation indicates part by weight (%). The term "balance" in the formulation indicates the remainder of the component amount.

**Formulation 1 (Granules)**

| | |
|---|---|
| Compound according to the present invention | 5% |
| Kaolin | 94% |
| White carbon | 1% |

The compound according to the present invention is dissolved in an organic solvent, and then sprayed onto a carrier, followed by evaporating the solvent under reduced pressure. These kinds of granules may be mixed with animal feed.

**Formulation 2 (Granules)**

| | |
|---|---|
| Compound according to the present invention | 10% |
| Attapulgite | 90% |

**Formulation 3 (Granules)**

| | |
|---|---|
| Compound according to the present invention | 3% |
| Polyethylene glycol | 3% |
| Kaolin | 94% |

The compound according to the present invention is finely pulverized, and then kneaded with kaolin preliminarily wetted with polyethylene glycol to obtain a formulation having a granular surface coated with the compound according to the present invention.

**Formulation 4 (Injection agent)**

| | |
|---|---|
| Compound according to the present invention | 0.1 to 1% |
| Peanut oil | Balance |

Afer preparation, the resultant is sterilized by filtration using a sterilizing filter.

**Formulation 5 (Injection agent)**

| | |
|---|---|
| Compound according to the present invention | 0.1 to 1% |
| Sesame oi | Balance |

**Formulation 6 (Pour-on agent)**

| | |
|---|---|
| Compound according to the present invention | 5% |
| Myristic acid ester | 10% |
| Isopropanol | Balance |

**Formulation 7 (Pour-on agent)**

| | |
|---|---|
| Compound according to the present invention | 2% |
| Medium-chain triglycerides | 15% |
| Ethanol | Balance |

**Formulation 8 (Pour-on agent)**

| | |
|---|---|
| Compound according to the present invention | 2% |
| Oleic acid ester | 5% |
| NMP | 40% |
| Isopropanol | Balance |

**Formulation 9 (Spot-on agent)**

| | |
|---|---|
| Compound according to the present invention | 10 to 15% |
| Ethyl carbitol | Balance |

**Formulation 10 (Spot-on agent)**

| | |
|---|---|
| Compound according to the present invention | 10 to 15% |
| Palmitic acid ester | 10% |
| Isopropanol | Balance |

**Formulation 11 (Spot-on agent)**

| | |
|---|---|
| Compound according to the present invention | 10 to 15% |
| Isopropanol | 20% |
| Benzyl alcohol | Balance |

**Formulation 12 (Spray-on agent)**

| | |
|---|---|
| Compound according to the present invention | 1% |
| Isopropanol | 40% |
| Propylene carbonate | Balance |

**Formulation 13 (Spray-on agent)**

| | |
|---|---|
| Compound according to the present invention | 1% |
| Propylene glycol | 10% |
| Isopropanol | Balance |

**Formulation 14 (liquid agent)**

| | |
|---|---|
| Compound according to the present invention | 5% |
| Propylene glycol | 15% |
| NMP | 65% |
| Water | Balance |

### EXAMPLES

The following test examples show that the compound according to the present invention is useful as an active ingredient of a parasite-control or parasiticide agent to be used to treat parasitic diseases.

First, examples of the amide compound (I) are shown in Tables 1 to 4.
Substituents of compounds represented by formula (a) are shown in Table 1.
Substituents of compounds represented by formula (b) are shown in Table 2.
Substituents of compounds represented by formula (c) are shown in Table 3.
Substituents of compounds represented by formula (d) are shown in Table 4.

In the tables, Me represents a methyl group, Et represents an ethyl group, ⁿPr represents a normal propyl group, ⁱPr represents an iso propyl group, ⁿBu represents a normal butyl group, ^{s}Bu represents a secondary butyl group, ^{t}Bu represents a tertiary butyl group, and ⁱBu represents an iso butyl group.

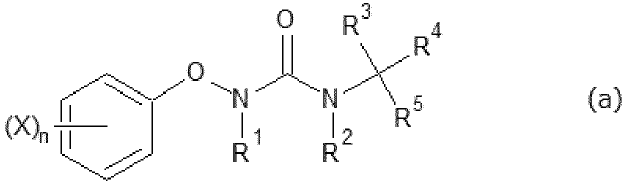

**Table 1**

| No. | R¹ | R² | R³ | R⁴ | R⁵ | (X)n | Physical properties |
|---|---|---|---|---|---|---|---|
| a-1 | Et | H | Me | Me | Phenyl | 3-Br-5-Cl | m.p.92-93°C |
| a-2 | Et | H | Me | Me | Phenyl | 3-Br-5-CF₃ | m.p.112-114°C |
| a-3 | Et | H | Me | Me | Phenyl | 3-Br-4,5-Cl₂ | m.p.100-102°C |
| a-4 | Et | H | Me | Me | Pyridin-2-yl | 3-Br-5-Cl | m.p.130-131°C |
| a-5 | Et | H | Me | Me | Pyridin-2-yl | 3,5-Br₂-4-Cl | ***** |
| a-6 | Et | H | Me | Me | 4-Me-Pyridin-2-yl | 3-Br-5-Cl | m.p.68-70°C |
| a-7 | Et | H | Me | Me | Pyridin-2-yl | 4-Br-3,5-Cl₂ | m.p.117-119°C |
| a-8 | Et | H | Me | Me | CONHⁱPr | 3-Br-5-Cl | * |
| a-9 | Et | H | Me | Me | CONHCH₂CF₃ | 3-Br-5-Cl | m.p.129-131°C |
| a-10 | Et | H | Me | Me | CONHCH₂C≡CH | 3-Br-5-Cl | * |
| a-11 | Et | H | Me | Me | CONHCH₂CF₃ | 4-Br-3.5-Cl₂ | ***** |
| a-12 | Et | H | Me | Me | Pyridin-2-yl | 3,4-Cl₂ | n_{D}(20.2°C) 1.5458 |
| a-13 | Et | H | Me | CN | Pyridin-2-yl | 3-Br-5-Cl | m.p.164-165°C |
| a-14 | Et | H | Me | Me | Pyridin-2-yl | 3,4,5-Cl₃ | m.p.92-94°C |
| a-15 | Et | H | Me | Me | Pyridin-2-yl | 3-Cl-5-CN | m.p.130-132°C |
| a-16 | Et | H | Me | Me | Pyridin-2-y) | 3,4-Br₂-5-Cl | m.p.104-106°C |
| a-17 | Et | H | Me | Me | 4-Phenyl-Pridin-2-yl | 3-Br-5-Cl | m.p.140-142°C |
| a-18 | Et | H | Me | Me | 5-F-Pyridin-2-yl | 3-Br-5-Cl | m.p.98-100°C |
| a-19 | Et | H | Me | Me | Pyrimidin-2-yl | 3-Br-5-Cl | m.p.137-138°C |
| a-20 | Et | H | Me | Me | Pyrimidin-4-yl | 3-Br-5-Cl | * |
| a-21 | Et | H | Me | Me | 4,5-Me₂-thiazol-2-yl | 3-Br-5-Cl | m.p.73-76°C |
| a-22 | Et | H | Me | Me | 4-SO₂Et-Pyridin-2-yl | 3-CN | m.p.100-103°C |
| a-23 | Et | H | Me | Me | 1-Et-1H-[1.2,4]triazol-3-yi | 3-Br-5-Cl | m.p.116-118°C |
| a-24 | Et | H | Me | Me | 2-Me-2H-tetrazol-5-yl | 3-Br-5-Cl | * |
| a-25 | Et | H | Me | Me | Pyrimidin-2-yl | 4-Br-3,5-Cl₂ | m.p.127-128°C |
| a-26 | Et | H | Me | Me | 4-SMe-Pyridin-2-yl | 3-Br-5-Cl | ***** |
| a-27 | Et | H | Me | Me | 4-SO₂Me-Pyridin-2-yl | 3-Br-5-Cl | m.p.93-95°C |
| a-28 | Et | H | Me | Me | 4-SMe-Pyridin-2-yl | 4-Br-3,5-Cl₂ | m.p.120-121°C |
| a-29 | Et | H | Me | Me | 4-SO₂Me-Pyridin-2-yl | 4-Br-3,5-Cl₂ | m.p.135-136°C |
| a-30 | Et | H | Me | Me | 4-SCH₂CF₃-Pyridin-2-yl | 3-Br-5-Cl | m.p.107-108°C |
| a-31 | Et | H | Me | Me | 4-SOEt-Pyridin-2-yl | 3-Cl-4,5-Br₂ | m.p.143-144°C |
| a-32 | Et | H | Me | Me | 4-NMe₂-Pyrimidin-2-yl | 3-Br-5-Cl | m.p.83-84°C |
| a-33 | Et | H | Me | Me | 4-N=S(=O)Me₂-Pyrimidin-2-yl | 3-Br-5-Cl | m.p.166-167°C |
| a-34 | Et | H | Me | Me | Pyrimidin-2-yl | 3-Cl-4,5-Br₂ | m.p.112-114°C |
| a-35 | Et | H | Me | Me | 4-^{t}Bu-Pyridin-2-yl | 3-Br-5-Cl | m.p.96-98°C |
| a-36 | Et | H | Me | Me | Pyrimidin-2-yl | 3-Br-4,5-Cl₂ | viscous oil |
| a-37 | Et | H | Me | Me | 4-(Pyridin-4-yl)-Pyridin-2-yl | 3-Br-5-Cl | m.p.134-136°C |
| a-38 | Et | H | Me | Me | 4-ⁱPr-Pyridin-2-yl | 3-Br-5-Cl | n_{D}(20.9°C) 1.5557 |
| a-39 | Et | H | Me | Me | CONHCH₂CN | 3-Br-5-Cl | m.p.133-135°C |
| a-40 | Et | H | Me | Me | Pyrazin-2-yl | 3-Br-5-Cl | n_{D}(20.5°C) 1.5400 |
| a-41 | Et | H | Me | Me | 4-CF₃-Pyridin-2-yl | 3-Br-5-Cl | m.p.74-76°C |
| a-42 | Et | H | Me | Me | 4-Me-thiazol-2-yl | 3-Br-5-Cl | m.p.94-96°C |
| a-43 | Et | H | Me | Me | 1-Me-1H-pyrazol-3-yl | 3-Br-5-Cl | n_{D}(20.5°C)1.5310 |
| a-44 | Et | H | Me | Me | [1,2,4]Triazin-3-yl | 3-Br-5-Cl | m.p.100-101°C |
| a-45 | Et | H | Me | Me | 4-Me-Pyrimidin-2-yl | 3-Br-5-Cl | viscous oil |
| a-46 | Et | H | Me | Me | 4-(o-tolyl)pyridin-2-yl | 3-Br-5-Cl | amorphous |
| a-47 | Et | H | Me | Me | 4-F-Pyridin-2-yl | 3-Br-5-Cl | viscous oil |
| a-48 | Et | H | Me | Me | 4-(thiophen-2-yl)pyridin-2-yl | 3-Br-6-Cl | m.p.100-102°C |
| a-49 | Et | H | Me | Me | 4-(pyridin-4-yl)pyrimidin-2-yl | 4-Cl-3,5-F₂ | n_{D}(20.1°C)1.5739 |
| a-50 | Et | H | Me | Me | [2,4'-bipyridin]-2'-yl | 3-Br-5-Cl | m.p.132-134°C |
| a-51 | Et | H | Me | Me | [3,4-bipyridin]-2'-yl | 3-Br-5-Cl | m.p.126-128°C |
| a-52 | Et | H | Me | Me | CONHCH₂CH₂SMe | 3-Br-5-Cl | viscous oil |
| a-53 | Et | H | Me | Me | CONHC(Me)₂CN | 3-Br-5-Cl | m.p.131-133°C |
| a-54 | Et | H | Me | Me | CONHCH₂CH₂CN | 3-Br-5-Cl | m.p.134-136°C |
| a-55 | Et | H | Me | Me | 4-SEt-Pyridin-2-yl | 3-CN | viscous oil |

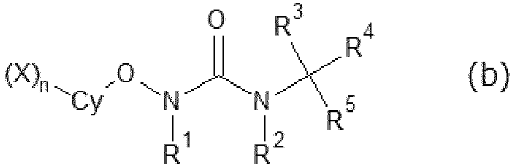

**Table 2**

| No. | R¹ | R² | R³ | R⁴ | R⁵ | Cy | (X)n | physical properties |
|---|---|---|---|---|---|---|---|---|
| b-1 | Et | H | Me | Me | Me | Quinolin-6-yl | 3-Br | m.p.85-89°C |
| b-2 | Et | H | Me | Me | C≡CMe | Quinolin-6-yl | 3-Br | m.p.86-88°C |
| b-3 | Et | H | Me | Me | Phenyl | Quinolin-6-yl | 3-Br | m.p.115-116°C |
| b-4 | Et | H | Me | Me | Pyridin-2-yl | Quinolin-6-yl | 3-Br | m.p.110-111°C |
| b-5 | Et | H | Me | Me | Phenyl | Pyridin-2-yl | 4-CF₃-6-Cl | m.p.97-99°C |
| b-6 | Et | H | Me | Me | Pyridin-2-yl | Pyridin-2-yl | 4-CF₃-6-Cl | m.p.90-99°C |
| b-7 | Et | H | Me | Me | 4-SO₂Et-Pyridin-2-yl | Pyridin-4-yl | 2-CN | m.p.122-124°C |
| b-8 | Et | H | Me | Me | Pyridin-2-yl | Pyridin-4-yl | 2,6-Cl₂ | m.p.87-89°C |
| b-9 | Et | H | Me | Me | Pyrimidin-2-yl | Pyridin-2-yl | 4-CF₃-6-Cl | n_{D}(20.8°C) 1.5101 |
| b-10 | Et | H | Me | Me | Pyridin-2-yl | Pyridin-4-yl | 2,6-Br₂ | m.p.136-138°C |
| b-11 | Et | H | Me | Me | Pyrimidin-2-yl | Pyridin-4 yl | 2,6-Cl₂ | m.p.140-141°C |
| b-12 | Et | H | Me | Me | Pyridin-2-yl | Pyridin-4-yl | 2-Cl-6-N(Me)₂ | m.p.84-86°C |
| b-13 | Et | H | Me | Me | Pyridin-2-yl | Thiazolo[4,5-c]pyridin-6-yl | 2-CF₃ | m.p.120-122°C |
| b-14 | Et | H | Me | Me | 4-SO₂Me-Pyridin-2-yl | Pyridin-4-yl | 2-Cl-6-CF₃ | m.p.149-150°C |
| b-15 | Et | H | Me | Me | 4-SMe-Pyridin-2-yl | Pyridin-2-yl | 4-CF₃-6-Cl | m.p.64-67°C |
| b-16 | Et | H | Me | Me | 4-SO₂Me-Pyridin-2-yl | Pyridin-2-yl | 4-CF₃-6-Cl | m.p.110-112°C |
| b-17 | Et | H | Me | Me | 4-SMe-Pyridin-2-yl | Pyridin-4-yl | 2,6-Br₂ | m.p.133-134°C |
| b-18 | Et | H | Me | Me | 4-SO₂Me-Pyridin-2-yl | Pyridin-4-yl | 2,6-Br₂ | m.p.160-161°C |
| b-19 | Et | H | Me | Me | 4-SEt-Pyridin-2-yl | Pyridin-2-yl | 4-CF₃-6-Cl | m.p.59-61°C |
| b-20 | Et | H | Me | Me | 4-SEt-Pyridin-2-yl | Pyridin-4-yl | 2,6-Br₂ | n_{D}(20.7°C) 1.5858 |
| b-21 | Et | H | Me | Me | 4-SⁱPr-Pyridin-2-yl | Pyridin-2-yl | 4-CF₃-6-Cl | n_{D}(20.9°C) 1.5247 |
| b-22 | Et | H | Me | Me | 4-SO2ⁱPr-Pyridin-2-yl | Pyridin-2-yl | 4-CF₃-6-Cl | m.p.126-127 |
| b-23 | Et | H | Me | Me | 4-SO₂Et-Pyridin-2-yl | Pyridin-2-yl | 4-CF₃-6-Cl | m.p.98-100°C |
| b-24 | Et | H | Me | Me | 4-SMe-Pyridin-2-yl | Pyridin-4-yl | 2,6-Cl₂ | ***** |
| b-25 | Et | H | Me | Me | 4-SO₂Me-Pyridin-2-yl | Pyridin-4-yl | 2,6-Cl₂ | * |
| b-26 | Et | H | Me | Me | 4-SMe-Pyridin-2-yl | Pyridin-4-yl | 2-Cl-6-N(Me)₂ | * |
| b-27 | Et | H | Me | Me | 4-SOMe-Pyridin-2-yl | Pyridin-4-yl | 2-Cl-6-N(Me)₂ | m.p.100-102°C |
| b-28 | Et | H | Me | Me | 4-SO₂Me-Pyridin-2-yl | Pyridin-2-yl | 4-CF₃-6-Me | m.p.132-133°C |
| b-29 | Et | H | Me | Me | 4-OCH₂CF₃-Pyridin-2-yl | Pyridin-4-yl | 2-CN | m.p.82-84°C |
| b-30 | CH₂C≡CH | H | Me | Me | 4-SMe-Pyridin-2-yl | Pyridin-2-yl | 4-CF₃-6-Cl | * |
| b-31 | Me | H | Me | Me | 4-SMe-Pyridin-2-yl | Pyridin-2-yl | 4-CF₃-6-Cl | m.p.70-72°C |
| b-32 | Et | H | Me | Me | 4-N(Me)₂-Pyrimidin-2-yl | Pyridin-2-yl | 4-CF₃-6-Cl | m.p.130-131°C |
| b-33 | Et | H | Me | Me | Pyrimidin-2-yl | Pyridin-4-yl | 2,6-Br₂ | m.p.166-168°C |
| b-34 | Et | H | Me | Me | 5-F-Pyridin-2-yl | Pyridin-4-yl | 2,6-Br₂ | m.p.122-124°C |
| b-35 | Et | H | Me | Me | 5-F-Pyridin-2-yl | Pyridin-2-yl | 4-CF₃-6-Cl | viscous oil |
| b-36 | Et | H | Me | Me | Pyridin-2-yl | Pyridin-2-yl | 4-CN-6-Cl | viscous oil |
| b-37 | Et | H | Me | Me | Pyridin-2-yl | Pyridin-4-yl | 2-Cl | n_{D}(23.8°C)1.5248 |
| b-38 | Et | H | Me | Me | Pyridin-2-yl | Pyridin-4-yl | 2-CN-6-Me | viscous oil |
| b-39 | Et | H | Me | Me | Pyridin-2-yl | Pyridin-3-yl | 5,6-Cl₂ | m.p.90-92°C |
| b-40 | Et | H | Me | Me | 4-Phenyl-Pyridin-2-yl | Pyridin-4-yl | 2,6-Cl₂ | amorphous |
| b-41 | Et | H | Me | Me | 4-SⁱPr-Pyridin-2-yl | Pyridin-4-yl | 2,6-Br₂ | m.p.96-97°C |

| No. | R¹ | R² | R³ | R⁴ | R⁵ | Cy | (X)n | Physical properties |
|---|---|---|---|---|---|---|---|---|
| b-42 | Et | H | Me | Me | 4-ⁱPr-Pyridin-2-yl | Pyridin-4-yl | 2,6-Br₂ | n_{D}(20.9°C)1.5565 |
| b-43 | Et | H | Me | Me | 4-ⁱPr-Pyridin-2-yl | Pyridin-2-yl | 6-Cl-4-CF₃ | n_{D}(21.4°C)1.5078 |
| b-44 | Et | H | Me | Me | 4-(1,2-dimethylhydrazinyl)Pyrimidin-2-yl | Pyridin-2-yl | 6-Cl-4-CF₃ | ***** |
| b-45 | Et | H | Me | Me | Pyrimidin-2-yl | Pyridin-4-yl | 2-Cl-6-NMe₂ | n_{D})(18.8°C)1.5508 |
| b-46 | Et | H | Me | Me | 4-SO₂Me-Pyridin-2-yl | Pyridin-4-yl | 2-Cl-6-NMe₂ | ***** |
| b-47 | Et | H | Me | Me | Pyrimidin-2-yl | Pyridin-4-yl | 2-Br-6-Cl | m.p.111-113°C |
| b-48 | Et | H | Me | Me | 4-SEt-Pyridin-2-yl | Pyridin-4-yl | 2-Br-6-Cl | * |
| b-49 | Et | H | Me | Me | 4-SO₂Et-Pyridin-2-yl | Pyridin-4-yl | 2-Br-6-Cl | m.p.121-123°C |
| b-50 | Et | H | Me | Me | 4-SⁿPr-Pyridin-2-yl | Pyridin-4-yl | 2-Br-6-Cl | m.p.98-99°C |
| b-51 | Et | H | Me | Me | CONHOH₂CN | Pyridin-2-yl | 6-Cl-4-CF₃ | m.p.158-160°C |
| b-52 | Et | H | Me | Me | 4-SⁿBu-Pyridin-2-yl | Pyridin-2-yl | 6-Cl-4-CF₃ | ***** |
| b-53 | Et | H | Me | Me | Pyridin-2-yl | Pyridin-2-yl | 6-Cl-4-CHF₂ | viscous oil |
| b-54 | Et | H | Me | Me | 4-SMe-Pyridin-2-yl | Pyridin-2-yl | 6-Cl-4-CHF₂ | ***** |
| b-55 | Et | H | Me | Me | 4-SO₂Me-Pyridin-2-yl | Pyridin-2-yl | 6-Cl-4-CHF₂ | m.p.128-18°C |
| b-56 | Et | H | Me | Me | 4-S(=O)Et-Pyridin-2-yl | Pyridin-2-yl | 6-Cl-4-CF₃ | * |
| b-57 | Et | H | Me | Me | 4-SEt-Pyridin-2-yl | Pyridin-2-yl | 6-Cl-4-CF₂Me | m.p.124-126°C |
| b-58 | Et | H | Me | Me | 4-SCH₂CF₃-Pyridin-2-yl | Pyridin-2-yl | 6-Cl-4-CF₃ | m.p.63-65°C |
| b-59 | Et | H | Me | Me | 4-SCH₂CF₃-Pyridin-2-yl | Pyridin-4-yl | 2,6-Cl₂ | m.p.138-139°C |
| b-60 | Et | H | Me | Me | 4-S(=O)CH₂CF₃-Pyridin-2-yl | Pyridin-4-yl | 2,6-Cl₂ | m.p.140-141°C |
| b-61 | Et | H | Me | Me | 2-Et-2*H*-tetrazol-5-yl | Pyridin-2-yl | 6-Cl-4-CF₃ | amorphous |
| b-62 | Et | H | Me | Me | 2-Me-2*H*-tetrazol-5-yl | Pyridin-2-yl | 6-Cl-4-CF₃ | n_{D}(20.6°C)1.4966 |
| b-63 | Et | H | Me | Me | 2-ⁿPr-2*H*-tetrazol-5-yl | Pyridin-2-yl | 6-Cl-4-CF₃ | n_{D}(20.6°C)1.4941 |
| b-64 | Et | H | Me | Me | 2-CH₂CF₃-2*H*-tetrazol-5-yl | Pyridin-2-yl | 6-Cl-4-CF₃ | m.p.87-89°C |
| b-65 | Et | H | Me | Me | 4-OCH₂CF₃-Pyridin-2-yl | Pyridin-2-yl | 6-Cl-4-CF₃ | n_{D}(21.3°C)1.4932 |
| b-66 | Et | H | Me | Me | 4-OCH₂CF₃-2-Pyridin-yl | Pyridin-4-yl | 2,6-Cl₂ | viscous oil |
| b-67 | Et | H | Me | Me | 4-SEt-Pyridin-2-yl | Pyridin-4-yl | 2-Cl-6-CF₃ | viscous oil |
| b-68 | Et | H | Me | Me | 4-S(=O)Et-Pyridin-2-yl | Pyridin-4-yl | 2-Cl-6-CF₃ | m.p.112-113°C |
| b-69 | Et | H | Me | Me | 4-SO₂Et-Pyridin-2-yl | Pyridin-4-yl | 2-Cl-6-CF₃ | m.p.111-112°C |
| b-70 | Et | H | Me | Me | 4-⁵Bu-Pyridin-2-yl | Pyridin-2-yl | 6-Cl-4-CF₃ | n_{D}(24.9°C)1.5060 |
| b-71 | Et | H | Me | Me | 4-OCH₂CF₃-Pyridin-2-yl | Pyridin-4-yl | 2-Br-6-Cl | m.p.116-117°C |
| b-72 | Et | H | Me | Me | 4-SEt-Pyridin-2-yl | Pyridin-2-yl | 4-CN | m.p.100-101°C |
| b-73 | Et | H | Me | Me | 4-OCH₂CHF₂-Pyridin-2-yl | Pyridin-2-yl | 6-Cl-4-CF₃ | n_{D}(20.3°C)1.5019 |
| b-74 | Et | H | Me | Me | 4-OCH₂CHF₂-Pyridin-2-yl | Pyridin-4-yl | 2,6-Cl₂ | n_{D}(20.6°C)1.5327 |
| b-75 | Et | H | Me | Me | 4-SO₂Et-Pyridin-2-yl | Pyridin-2-yl | 4-CN | m.p.97-99°C |
| b-76 | Et | H | Me | Me | 4-SEt-Pyridin-2-yl | Pyridin-4-yl | 2-CN | viscous oil |
| b-77 | Et | H | Me | Me | 4-S(=O)Et-Pyridin-2-yl | Pyridin-4-yl | 2-CN | m.p.101-103°C |

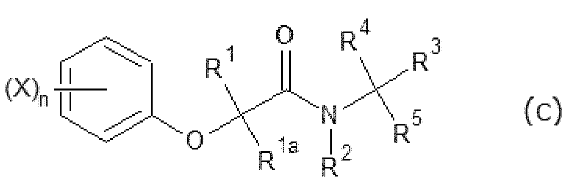

**Table 3**

| No. | R¹ | R^{1a} | R² | R³ | R⁴ | R⁵ | (X)n | Physical properties |
|---|---|---|---|---|---|---|---|---|
| c-1 | Et | H | H | Me | Me | Pyridin-2-yl | 4-Br-3,5-Cl₂ | * |
| c-2 | Et | H | H | Me | Me | Pyrimidin-2-yl | 4-Br-3,5-Cl₂ | m.p.109-111°C |
| c-3 | Et | H | H | Me | Me | Pyrimidin-2-yl | 3,5-Br₂-4-Cl | m.p.121-123°C |
| c-4 | Et | H | H | Me | Me | Pyrimidin-2-yl | 3,4-Br₂-5-Cl | m.p.144-145°C |
| c-5 | Et | H | H | Me | Me | Pyridin-2-yl | 3-Br-5-Cl | m.p.110-112°C |
| c-6 | Et | H | H | Me | Me | 4-SMe-Pyridin-2-yl | 4-Cl-3,5-Br₂ | m.p.134-136°C |
| c-7 | Et | H | H | Me | Me | Pyridin-2-yl | 3-Cl-5-CN | m.p.90-92°C |
| c-8 | Et | H | H | Me | Me | Pyrimidin-2-yl | 3-Cl-5-CN | m.p.86-88°C |
| c-9 | Et | H | H | Me | Me | 4-SMe-Pyridin-2-yl | 3-Cl-5-CN | m.p.108-110°C |
| c-10 | Et | H | H | Me | Me | 5-F-Pyridin-2-yl | 4-Cl-3,5-Br₂ | m.p.112-114°C |
| c-11 | Et | H | H | Me | Me | Pyrimidin-2-yl | 3-Cl-5-CF₃ | m.p.81-83°C |
| c-12 | Et | H | H | Me | Me | 4-SEt-Pyridin-2-yl | 3-Cl-5-CN | m.p.114-116°C |
| c-13 | Et | H | H | Me | Me | 4-SO₂Et-Pyridin-2-yl | 3-Cl-5-CN | m.p.15-153°C |
| c-14 | Et | H | H | Me | Me | 4-SEt-Pyridin-2-yl | 3-Cl-5-CF₃ | m.p.74-76°C |
| c-15 | Et | H | H | Me | Me | 4-SO₂Et-Pyridin-2-yl | 3-Cl-5-CF₃ | m.p.119-121 °C |
| c-16 | Et | H | H | Me | Me | 4-SEt-Pyridin-2-yl | 3-CN-5-F | m.p.87-89°C |
| c-17 | Et | H | H | Me | Me | 4-SEt-Pyridin-2-yl | 3-Cl-5-OCF₃ | n_{D}(21.4°C)1.4729 |
| c-18 | Et | H | H | Me | Me | 4-SEt-Pyridin-2-yl | 3,5-(CF₃)₂ | m.p.96-98°C |
| c-19 | Et | H | H | Me | Me | 4-SO₂Et-Pyridin-2-yl | 3,5-(OF₃)₂ | m.p.139-141°C |
| c-20 | Et | H | H | Me | Me | 4-S(=O)Et-Pyridin-2-yl | 3,5-(CF₃)₂ | m.p.83-86°C |
| c-21 | Et | H | H | Me | Me | 4-SEt-Pyridin-2-yl | 3-CN | m.p.106-108°C |
| c-22 | Et | H | H | Me | Me | 4-SO₂Et-Pyridin-2-yl | 3-CN | m.p.114-116°C |
| c-23 | Et | H | H | Me | Me | 4-S(=O)Et-Pyridin-2-yl | 3-CN | viscous oil |
| c-24 | Et | H | H | Me | Me | 4-SEt-Pyridin-2-yl | 3,5-CN₂ | m.p.115-117°C |
| c-25 | Et | H | H | Me | Me | 4-SO₂Et-Pyridin-2-yl | 3,5-CN₂ | m.p.140-142°C |
| c-26 | Et | H | H | Me | Me | 4-OCH₂CF₃-2-Pyridin-yl | 3-Cl-5-CN | n_{D}(22.2°C)1.5226 |
| c-27 | Et | H | H | Me | Me | 4-SCH₂CF₃-Pyridin-2-yl | 3-Cl-5-CF₃ | m.p103-105°C |
| c-28 | Et | H | H | Me | Me | 4-SEt-Pyridin-2-yl | 3-F-5-CF₃ | m.p.70-71°C |
| c-29 | Et | H | H | Me | Me | 4-SEt-Pyridin-2-yl | 3-NO₂ | m.p.87-89°C |
| c-30 | Et | H | H | Me | Me | 4-OCH₂CF₃-Pyridin-2-yl | 3-CN | n_{D}(24.7°C)1.5089 |
| c-31 | Et | H | H | Me | Me | 4-SEt-Pyridin-2-yl | 3-(CH=N-OMe) | m.p.61-63°C |
| c-32 | Et | H | H | Me | Me | 4-SCH₂CF₃-Pyridin-2-yl | 3-CN-5-F | m.p.135-136°C |
| c-33 | Et | H | H | Me | Me | 4-SCH₂CF₃-Pyridin-2-yl | 3-Cl-5-CN | m.p.161-162°C |
| c-34 | Et | H | H | Me | Me | 4-SEt-Pyridin-2-yl | 4-Cl-3-CN | m.p.100-102°C |
| c-35 | Et | H | H | Me | Me | 4-OCH₂CHF₂-Pyridin-2-yl | 3-CN-5-F | n_{D}(20.0°C)1.5188 |
| c-36 | Et | H | H | Me | Me | 4-(1*H*-imidazol-1-yl)Pyrimidin-2-yl | 3-CN | m.p.156-158°C |
| c-37 | Et | H | H | Me | Me | 4-(N=S(=O)Me₂)Pyrimidin-2-yl | 3-CN | n_{D}(20.0°C)1.5505 |

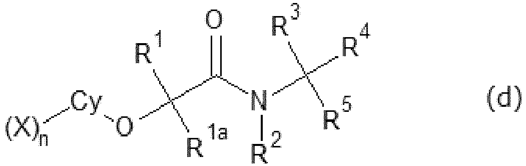

**Table 4**

| No. | R¹ | R^{1a} | R² | R³ | R⁴ | R⁵ | Cy | (X)n | Physical properties |
|---|---|---|---|---|---|---|---|---|---|
| d-1 | Et | H | H | Me | Me | Pyridin-2-yl | Quinolin-6-yl | - | m.p.111-113°C |
| d-2 | Et | H | H | Me | Me | 4-SMe-Pyridin-2-yl | Pyridin-4-yl | 2,6-Cl₂ | * |
| d-3 | Et | H | H | Me | Me | 4-SO₂Me-Pyridin-2-yl | Pyridin-4-yl | 2,6-Cl₂ | m.p.179-181°C |
| d-4 | Et | H | H | Me | Me | CONHCH₂CF₃ | Pyridin-4-yl | 2,6-Cl₂ | amorphous |
| d-5 | Et | H | H | Me | Me | 4-SEt-Pyridin-2-yl | Pyridin-3-yl | 5-CN | m.p.91-92°C |
| d-6 | Et | H | H | Me | Me | 4-SO₂Et-Pyridin-2-yl | Pyridin-3-yl | 5-CN | m.p.107-108°C |
| d-7 | Et | H | H | Me | Me | 4-OCH₂CF₃-Pyridin-2-yl | Pyridin-3-yl | 5-CN | m.p.109-110°C |

¹H-NMR (CDCl₃) data of the compounds indicated by * in the colum of physical properties are shown separately.

a-5: ¹H-NMR (CDCl₃ /TMS, δ(ppm)) 8.44 (ddd, 1H), 8.16 (br, 1H), 7.70 (dt, 1H), 7.55 (s, 2H), 7.37 (dd, 1H), 7.17 (ddd, 1H), 3.68 (q, 2H), 1.74 (s, 6H), 1.19 (t, 3H).
a-8: ¹H-NMR (CDCl₃ /TMS, δ(ppm)) 7.24-7.22 (m, 2H), 7.12 (t, 1H), 6.42 (br, 1H), 5.98 (br, 1H), 4.08-3.96 (m, 1H), 3.63 (q, 2H), 1.56 (s, 6H), 1.18-1.13 (m, 9H).
a-10: ¹H-NMR (CDCl₃/TMS, δ(ppm)) 7.24-7.21 (m, 2H), 7.11 (t, 1H), 6.64 (br, 1H), 6.17 (br, 1H), 4.07-4.04 (m, 2H), 3.64 (q, 2H), 2.25-2.23 (m, 1H), 1.57 (s, 6H), 1.16 (t, 3H).
a-11: ¹H-NMR (CDCl₃ /TMS, δ(ppm)) 7.21 (s, 2H), 7.08 (br, 1H), 5.95 (br, 1H), 3.97-3.90 (m, 1H), 3.64 (q, 2H), 1.55 (s, 6H), 1.14 (t, 3H).
a-20: ¹H-NMR (CDCl₃/TMS, δ(ppm)) 9.12 (s, 1H), 8.71 (d, 1H), 7.39 (d, 1H), 7.34-7.32 (m, 1H), 7.26-7.21 (m, 2H), 7.13 (br, 1H), 3.64 (q, 2H), 1.71 (s, 6H), 1.17 (t, 3H).
a-24: ¹H-NMR (CDCl₃ /TMS, δ(ppm)) 7.30 (dd, 1H), 7.24 (t, 1H), 7.19 (t, 1H), 6.36 (br, 1H), 4.32 (s, 3H), 3.61 (q, 2H), 1.79 (s, 6H), 1.15 (t, 3H).
a-26: ¹H-NMR (CDCl₃/TMS, δ(ppm)) 8.23 (d, 1H), 7.92 (br, 1H), 7.31 (d, 1H), 7.20-7.18 (m, 2H), 7.13 (d, 1H), 6.95 (dd, 1H), 3.65 (t, 2H), 2.47 (s, 3H), 1.71 (s, 6H), 1.17 (t, 3H).
b-24: ¹H-NMR (CDCl₃/TMS, δ(ppm)) 8.20 (d, 1H), 8.14 (s, 1H), 7.17 (s, 2H), 7.15 (d, 1H), 6.99 (dd, 1H), 3.70 (br, 2H), 2.50 (s, 3H), 1.72 (s, 6H), 1.19 (t, 3H).
b-25: ¹H-NMR (CDCl₃/TMS, δ(ppm)) 8.76 (d, 1H), 7.89 (s, 1H), 7.70 (dd, 1H), 7.30 (s, 1H), 7.17 (s, 2H), 3.66 (br, 2H), 3.11 (s, 3H), 1.77 (s, 6H), 1.18 (t, 3H).
b-26: ¹H-NMR (CDCl₃ /TMS, δ(ppm)) 8.23 (d, 1H), 7.81 (s, 1H), 7.14 (d, 1H), 6.96 (dd ,1H), 6.46 (d, 1H), 6.21 (d, 1H), 3.67 (br, 2H), 3.06 (s, 6H), 2.48 (s, 3H), 1.71 (s, 6H), 1.18 (t, 3H).
b-30: ¹H-NMR (CDCl₃/TMS, δ(ppm)) 8.27 (s, 1H), 8.24 (d, 1H), 7.46 (s, 1H), 7.33 (s, 1H), 7.17 (d, 1H), 6.98 (dd, 1H), 4.48 (d, 2H), 2.49 (s, 3H), 2.24 (t, 1H), 1.74 (s, 6H).
b-44: ¹H-NMR (CDCl₃, δ(ppm)) 8.34 (s, 1H), 8.14 (d, 1H), 7.46 (s, 1H), 7.29 (s, 1H), 6.75 (br, 1H), 3.77 (br, 2H), 3.23 (s, 3H), 2.64 (s, 3H), 1.76 (s, 6H), 1.23 (t, 3H).
b-46: ¹H-NMR (CDCl₃, δ(ppm)) 8.76 (d, 1H), 7.87 (s, 1H), 7.65 (dd, 1H), 6.94 (s, 1H), 6.44 (d, 1H), 6.19 (d, 1H), 3.63 (br, 2H), 3.08 (s, 9H), 1.75 (s, 6H), 1.17 (t, 3H).
b-48: ¹H-NMR (CDCl₃, δ(ppm)) 8.21 (s, 1H), 8.19 (d, 1H), 7.34 (d, 1H), 7.20 (d, 1H), 7.14 (d, 1H), 6.99 (dd, 1H), 3.70 (br, 2H), 3.01 (q, 2H), 1.72 (s, 6H), 1.39 (t, 3H), 1.19 (t, 3H).
b-52: ¹H-NMR (CDCl₃, δ(ppm)) 8.21 (d, 1H), 8.06 (s, 1H), 7.42 (s, 1H), 7.31 (s, 1H), 7.14 (d, 1H), 6.96 (dd, 1H), 3.75 (br, 2H), 2.96 (t, 2H), 1.71 (s, 6H), 1.73-1.65 (m, 2H), 1.55-1.42 (m, 2H), 1.23 (t, 3H), 0.96 (t, 3H).
b-54: ¹H-NMR (CDCl₃, δ(ppm)) 8.23 (d, 1H), 7.91 (s, 1H), 7.30 (s, 1H), 7.20 (s, 1H), 7.11 (d, 1H), 6.93 (dd, 1H), 6.57 (t, 1H), 3.72 (br, 2H), 2.46 (s, 3H), 1.69 (s, 6H), 1.20 (t, 3H).
b-56: ¹H-NMR (CDCl₃, δ(ppm)) 8.56 (d, 1H), 7.62 (s, 1H), 7.59 (s, 1H), 7.40 (s, 1H), 7.32-7.28 (m, 2H), 3.71 (br, 2H), 2.99-2.92 (m, 1H), 2.77-2.70 (m, 1H), 1.74 (s, 3H), 1.72 (s, 3H), 1.24 (t, 3H), 1.20 (t, 3H).
c-1: ¹H-NMR (CDCl₃ /TMS, δ(ppm)) 8.67 (br, 1H), 8.47-8.45 (m, 1H), 7.69 (dt, 1H), 7.33 (d, 1H), 7.17 (dd, 1H), 7.08 (s, 2H), 4.40 (dd, 1H), 2.03-1.98 (m, 2H), 1.73 (s, 3H), 1.69 (s, 3H), 1.04 (t, 3H).
d-2: ¹H-NMR (CDCl₃/TMS, δ(ppm)) 8.65 (br, 1H), 8.23 (d, 1H), 7.10 (d, 1H), 6.98 (dd, 1H), 6.88 (s, 2H), 4.51 (t, 1H), 2.48 (s, 3H), 2.11-1.99 (m, 2H), 1.71 (s, 3H), 1.66 (s, 3H), 1.04 (t, 3H).

### (Test Example 1) Measurement of bioactivity against Ascaridia galli and Oesophagostomum dentatum.

The bioactivity of the compound according to the present invention was investigated in vitro using two kinds of parasites parasitic in the gut-welling at the larval period; one of which was Ascaridia galli at the third larvae period ("L3"); and the other of which was Oesophagostomum dentatum at the third and fourth larvae period (respectively "L3" and "L4"). DMSO solutions containing the compound according to the present invention at various concentrations were prepared and incubated in 96-well microtiter plates. Then, 20 larval parasites per well were inoculated. The bioactivity was investigated by microscopic examination. The microscopic examination included evaluation of the mortality, damage, motility, progression of development, and neutral red uptake by the larval parasites in comparison with those of DMSO control. The bioactivity was defined by the minimum effective concentration ("MEC"), which is the concentration when at least one of the larval parasite shows mortality, damage, change in motility, change in progression of development, or no neutral red uptake. The compounds shown in Tables 1 to 4 exhibited activities against at least one kind of the target parasites at the MEC of 50 µM or less.

In addition, the compounds according to the present invention shown below exhibited activites against at least one kind of the target parasites at the MEC equivalent to that of levamisole.
a-1 to a-12, a-14 to a-45, a-47 to a-55, b-1 to b-29, b-31 to b-50, b-52 to b-77, c-2, c-3, c-5 to c-10, c-12 to c-37, d-1 to d-3, and d-5 to d-7.

### (Test Example 2) Effects on jirds infected with Haemonchus contortus.

Jirds were orally infected with 750 to 3000 Haemonchus contortus at the third larvae period. 10 days after the infection, an injection agent prepared by mixing the compound according to the present invention, DMF, and saline was subcutaneously administered once to the jirds at a dose of 10 mg of the compound according to the present invention per kg body weight (treated group). 5 days after the administration, the jirds were necropsied to count the number of the larval parasites in the stomach. During the test period, no side effects were observed in the jirds of the treated group. Effects were evaluated by the decreased number of the average larval parasites in the treated group in comparison with that in a control group in which jirds infected with Haemonchus contortus were not treated.

The compounds according to the present invention shown below exhibited at least an 80% decrease in the number of Haemonchus contortus.
a-15, a-19, a-39, b-16, b-33, and c-9.

### INDUSTRIAL APPLICABILITY

The parasite-control or parasiticide agent according to the present invention makes it possible to effectively and safely treat parasitic diseases caused by parasites that cause harm to humans and animals, particularly endoparasites such as nematodes, cestodes, and flukes.

## Claims

1. A parasite-control or parasiticide agent comprising at least one selected from the group consisting of compounds represented by formula (I) and salts thereof as an active ingredient thereof: 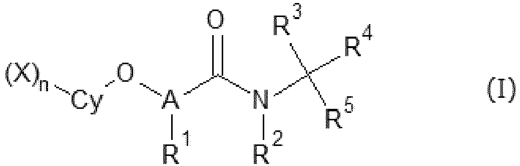 (in the formula (I), Cy represents a C6-10 aryl group or a heteroaryl group,
X represents a halogeno group, an unsubstituted or substituted C1-6 alkyl group, an unsubstituted or substituted C3-8 cycloalkyl group, an unsubstituted or substituted C2-6 alkenyl group, an unsubstituted or substituted C2-6 alkynyl group, a hydroxyl group, an unsubstituted or substituted C1-6 alkoxy group, an unsubstituted or substituted amino group, an unsubstituted or substituted C1-7 acyl group, an unsubstituted or substituted C1-6 alkoxycarbonyl group, an unsubstituted or substituted C1-6 alkylaminocarbonyl group, an unsubstituted or substituted C1-6 alkylthio group, an unsubstituted or substituted C1-6 alkylsulfinyl group, an unsubstituted or substituted C1-6 alkylsulfonyl group, an unsubstituted or substituted C6-10 aryl group, an unsubstituted or substituted heteroaryl group, a nitro group, or a cyano group,
n represents a number of X on Cy and is an integer of 0 to 5, and when n is 2 or more, X is identical or different,
R¹ represents an unsubstituted or substituted C1-6 alkyl group, an unsubstituted or substituted C2-6 alkenyl group, or an unsubstituted or substituted C2-6 alkynyl group,
A represents a group represented by CR^{1a} or a nitrogen atom (wherein R^{1a} represents a hydrogen atom, an unsubstituted or substituted C1-6 alkyl group, an unsubstituted or substituted C2-6 alkenyl group, or an unsubstituted or substituted C2-6 alkynyl group),
R² represents a hydrogen atom, an unsubstituted or substituted C1-6 alkyl group, an unsubstituted or substituted C2-6 alkenyl group, an unsubstituted or substituted C2-6 alkynyl group, an unsubstituted or substituted C1-7 acyl group, or an unsubstituted or substituted C1-6 alkoxycarbonyl group,
R³ and R⁴ each indenpendently represents a hydrogen atom, an unsubstituted or substituted C1-6 alkyl group, an unsubstituted or substituted C2-6 alkenyl group, an unsubstituted or substituted C2-6 alkynyl group, or a cyano group,
R⁵ represents an unsubstituted or substituted C1-6 alkyl group, an unsubstituted or substituted C2-6 alkenyl group, an unsubstituted or substituted C2-6 alkynyl group, an unsubstituted or substituted C1-7 acyl group, a carboxyl group, an unsubstituted or substituted C1-6 alkoxycarbonyl group, an unsubstituted or substituted C2-6 alkenyloxycarbonyl group, an unsubstituted or substituted C2-6 alkynyloxycarbonyl group, an aminocarbonyl group, an unsubstituted or substituted C1-6 alkylaminocarbonyl group, an unsubstituted or substituted C6-10 aryl group, or an unsubstituted or substituted heteroaryl group).

2. The parasite-control or parasiticide agent according to Claim 1, wherein R³ and R⁴ each indenpendently represents an unsubstituted or substituted C1-6 alkyl group, and
R⁵ represents an unsubstituted or substituted C6-10 aryl group, an unsubstituted or substituted heteroaryl group, or an unsubstituted or substituted C1-6 alkylaminocarbonyl group.

3. The parasite-control or parasiticide agent according to Claim 1 or 2, wherein R¹ represents an unsubstituted or substituted C1-6 alkyl group, and R² represents a hydrogen atom.

4. The parasite-control or parasiticide agent according to any one of Claims 1 to 3, wherein A represents a nitrogen atom.

5. The parasite-control or parasiticide agent according to any one of Claims 1 to 3, wherein A represents a group represented by CR^{1a}, and R^{1a} represents a hydrogen atom.

6. The parasite-control or parasiticide agent according to any one of Claims 1 to 5, wherein the parasite-control or parasiticide agent targets an endoparasite.

7. A method for controlling or expelling an endoparasite that is parasitic in warm-blooded animals or fishes, comprising administering a parasite-control or parasiticide agent of Cliam 6 to the warm-blooded animals or the fishes at an effective dose.

8. The parasite-control or parasiticide agent according to Claim 6, wherein the parasite-control or parasiticide agent targets nematodes.

9. A method for controlling or expelling nematodes that are parasitic in warm-blooded animals or fishes, comprising administering a parasite-control or parasiticide agent of Cliam 8 to the warm-blooded animals or the fishes at an effective dose.
